# EUROPÄISCHE PATENTSCHRIFT

(11) **EP 1 517 674 B1**
(45) Veröffentlichungstag und Bekanntmachung des Hinweises auf die Patenterteilung: **11.04.2007**
(21) Anmeldenummer: 03760605.0
(22) Anmeldetag: 12.06.2003
(51) Int. Cl.: A61K 9/08, A61K 38/05, A61K 9/00, A61P 25/06

(54) **ZUBEREITUNGEN ZUR INTRANASALEN APPLIKATION AUSGEWÄHLTER, VON AMINOSÄUREN ABGELEITETER CGRP-ANTAGONISTEN**
PREPARATIONS FOR THE INTRANASAL APPLICATION OF SELECTED CGRP ANTAGONISTS DERIVED FROM AMINO ACIDS AND A METHOD FOR THEIR PRODUCTION
PREPARATIONS POUR APPLICATION INTRANASALE D'ANTAGONISTES DE CGRP SELECTIONNES, DERIVES D'ACIDES AMINES, ET PROCEDE POUR LES PREPARER

(30) Priorität: 19.06.2002 DE 10227294
(43) Veröffentlichungstag der Anmeldung: 30.03.2005
(60) Teilanmeldung: 06123388.8
(73) Patentinhaber: Boehringer Ingelheim Pharma GmbH & Co. KG, 55216 Ingelheim am Rhein (DE)
(72) Erfinder: KRUSS, Bernd, 88454 Hochdorf (DE); GAISER, Marc, A., 88046 Friedrichshafen (DE); BUSCH, Ulrich, 88400 Biberach 1 (DE); JOST, Klaus, 88444 Ummendorf (DE)
(86) Internationale Anmeldenummer: PCT/EP2003/006156
(87) Internationale Veröffentlichungsnummer: WO 2004/000289

(56) Entgegenhaltungen:
- WO-A-01/10425
- WO-A-98/11128
- DE-A- 19 911 039

## Beschreibung

Die Erfindung betrifft pharmazeutische Zusammensetzungen für die nasale Applikation, umfassend ausgewählte CGRP-Antagonisten, die in der WO 98/11128 beschrieben werden, sowie ein Verfahren zu deren Herstellung.

Die nachstehend mit (A) bis (CJ) bezeichneten Verbindungen besitzen CGRP-antagonistische Eigenschaften und zeigen sehr gute Affinitäten in CGRP-Rezeptorbindungsstudien.

Folgende Verbindungen kommen, in Form ihrer Salze mit physiologisch verträglichen Säuren gelöst in Wasser, als Bestandteile der erfindungsgemäßen nasalen Applikationsformen in Frage:
(A) 1-[N²-[3,5-Dibrom-N-[[4-(3,4-dihydro-2(1 H)-oxochinazolin-3-yl)-1-piperidinyl]-carbonyl]-D-tyrosyl]-L-lysyl]-4-(4-pyridinyl)-piperazin,
(B) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,
(C) 1-[N²-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperazin ;
(D) 1-[N²-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperidin,
(E) 1-[N²-[3,5-Dibrom-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazin,
(F) 1-[N²-[4-Amino-3,5-dibrom-N-[[4-(1,3-dihydro-4-phenyl-2(2H)-oxoimidazol-1-yl)-1-pipieridinyl]carbonyl]-D-phenylalanyl]-L-lysyl]-4-(4-pyridinyl)-piperazin,
(G) 1-[3,5-Dibrom-N-[[4-(3,4-dihydro-2(1H)-oxothieno[3,4-d]pyrimidin-3-yl)-1-piperidinyl]carbonyl]-D-tyrosyl]-4-(1-piperidinyl)-piperidin,
(H) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidin,
(I) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,
(J) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,
(K) 1-[4-Amino-3,5-dibrom-N-[[4-(3,4-dihydro-2(1H)-oxothieno[3,2-d]pyrimidin-3-yl)-1-piperidinyl]carbonyl]-D-phenylalanyl]-4-(1-piperidinyl)-piperidin,
(L) 1-[4-Amino-3,5-dibrom-N-[[4-[1,3-dihydro-4-[3-(tri-fluoromethyl)phenyl]-2(2H)-oxoimidazol-1-yl]-1-piperidin-yl]carbonyl]-D-phenylalanyl]-4-(1-ethyl-4-piperidinyl)-piperidin,
(M) 1-[4-Amino-3,5-dibrom-N-[[4-[3,4-dihydro-2(1H)-oxochinazolin-3-yl]-1-piperidinyl]carbonyl]-D-phenylalanyl]-4-(1-hexyl-4-piperidinyl)-piperidin,
(N) 1-[4-Amino-3,5-dibrom-N-[[4-[3,4-dihydro-2(1H)-oxochinazolin-3-yl]-1-piperidinyl]carbonyl]-D-phenylalanyl]-4-(1-cyclopropylmethyl-4-piperidinyl)-piperidin,
(O) 1-[N-[[4-[3,4-Dihydro-2(1H)-oxochinazolin-3-yl]-1-piperidinyl]carbonyl]-3-ethenyl-D,L-phenylalanyl]-4-(hexahydro-1H-1-azepinyl)-piperidin,
(P) (R,S)-1-[4-[4-(3,4-Dihydro-2(1H)-oxochinazolin-3-yl)-1-piperidinyl]-2-[(4-hydroxy-3,5-dimethylphenyl)methyl]-1,4-dioxobutyl]-4-(1-piperidinyl)-piperidin,
(Q) 1-[4-Amino-3,5-dibrom-N-[[4-[N-(aminocarbonyl)-N-phenylamino]-1-piperidinyl]carbonyl]-D-phenylalanyl]-4-(1-piperidinyl)-piperidin,
(R) 1-[4-Amino-3,5-dibrom-N-[[4-(3,4-dihydro-2(1H)-oxochinazolin-3-yl)-1-piperidinyl]carbonyl]-D-phenylalanyl]-4-(5-methoxy-4-pyrimidinyl)-piperazin,
(S) 1-[4-Amino-3,5-dibrom-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidin
(T) 1-[4-Amino-3,5-dibrom-N-[[4-[2(1H)-oxochinolin-3-yl]-1-piperidinyl]carbonyl]-D-phenylalanyl]-4-(1-piperidinyl)-piperidin,
(U) 1-[4-Amino-3,5-dibrom-N-[[4-[3,4-dihydro-2(1H)-oxochinazolin-3-yl]-1-piperidinyl]carbonyl]-D-phenylalanyl]-4-[3-(dimethylamino)propyl]-piperazin,
(V) 1-[4-Amino-3,5-dibrom-N-[[4-[3,4-dihydro-2(1 H)-oxochinazolin-3-yl]-1-piperidinyl]carbonyl]-D-phenylalanyl]-4-(4-methyl-1-piperazinyl)-piperidin,
(W) 1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperidinyl)carbonyl]-piperazin,
(X) 1-[4-Amino-3,5-dibrom-N-[[4-[3,4-dihydro-2(1 H)-oxochinazolin-3-yl]-1-piperidinyl]carbonyl]-D-phenylalanyl]-4-[(1-methyl-4-piperazinyl)carbonyl]-piperazin,
(Y) 1-[4-Amino-3,5-dibrom-N-[[4-(3,4-dihydro-2(1H)-oxochinazolin-3-yl)-1-piperidinyl]carbonyl]-D-phenylalanyl]-4-[4-[4-(dimethylamino)butyl]phenyl]-piperazin,
(Z) 1-[4-Amino-3,5-dibrom-N-[[4-(3,4-dihydro-2(1H)-oxochinazolin-3-yl)-1 -piperidinyl]carbonyl]-D-phenylalanyl]-4-[4-(dimethylamino)-piperidinyl]-piperidin,
(AA) 1-[N²-[[4-(1,3-Dihydro-4-phenyl-2(2H)-oxoimidazol-1-yl)-1-piperidinyl]carbonyl]-N'-methyl-D-tryptyl]-4-(4-methyl-1-piperazinyl)-piperidin,
(AB) 1-[N²-[[4-(1,3-Dihydro-4-phenyl-2(2H)-oxoimidazol-1-yl)-1-piperidinyl]carbonyl]-N'-(1,1-dimethylethoxycarbonyl)-D-tryptyl]-4-(1-methyl-4-piperidinyl)-piperidin,
(AC) (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,
(AD) (R,S)-1-[4-[4-(3,4-Dihydro-2(1H)-oxochinazolin-3-yl)-1-piperidinyl]-2-[(3,5-dibrom-4-methoxyphenyl)methyl]-1,4-dioxobutyl]-4-(1-methyl-4-piperidinyl)-piperidin,
(AE) (R,S)-1-[4-[4-(3,4-Dihydro-2(1H)-oxochinazolin-3-yl)-1-piperidinyl]-2-[(3,4-dibromphenyl)methyl]-1,4-dioxobutyl]-4-(4-methyl-1-piperazinyl)-piperidin,
(AF) 1-[N²-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin,
(AG) 1-[4-Amino-3,5-dibrom-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidin,
(AH) 1-[N²-[4-Amino-3,5-dibrom-N-[[4-(1,3-dihydro-2(2H)-oxobenzimidazol-1-yl)-1-piperidinyl]carbonyl]-D-phenylalanyl]-N⁶,N⁶-dimethyl-L-lysyl]-4-(4-pyridinyl)-piperazin,
(AI) 1-[N²-[4-Amino-3,5-dibrom-N-[[4-(3,4-dihydro-2(1 H)-oxochinazolin-3-yl)-1-piperidinyl]carbonyf]-D-phenytalanyl]-N⁶,N⁶-dimethyl-L-lysyl]-4-(4-pyridinyl)-piperazin,
(AJ) (R,S)-1-[2-(4-Amino-3,5-dibrombenzoyl)-4-[4-(3,4-dihydro-2(1H)-oxochinazolin-3-yl)-1-piperidinyl]-4-oxobutyl]-4-(1-piperidinyl)-piperidin,
(AK) 1-[4-Amino-3,5-dibrom-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidin,
(AL) 1-[4-Amino-3,5-dibrom-N-[[4-[1,3-dihydro-2(2H)-oxoimidazo[4,5-c]chinolin-3-yl]-1-piperidinyl]carbonyl]-D-phenylalanyl]-4-(1-piperidinyl)carbonyl]-piperidin,
(AM) 1-[4-Amino-3,5-dibrom-N-[[4-(3,4-dihydro-2(1H)-oxochinazolin-3-yl)-1-piperidinyl]-carbonyl]-D-phenylalanyl]-4-(1-piperidinyl)-piperidin,
(AN) 1-[N²-[3,5-Dibrom-N-[[4-(3,4-dihydro-2(1H)-oxochinazolin-3-yl)-1-piperidinyl]-carbonyl]-D-tyrosyl]-N⁶,N⁶-dimethyl-L-lysyl]-4-(4-pyridinyl)-piperazin,
(AO) 1-[4-Amino-N-[[4-[4-(3-bromphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
(AP) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidin,
(AQ) 1-[4-Amino-3,5-dibrom-N-[[4-[1,3-dihydro-4-[3-(trifluormethyl)phenyl]-2(2H)-oxo-imidazol-1-yl]-1-piperidinyl]carbonyl]-D-phenylalanyl]-4-(1-piperidinyl)-piperidin,
(AR) 1-[4-Amino-3,5-dibrom-N-[[4-[1,3-dihydro-4-[3-(trifluormethyl)phenyl]-2(2H)-oxo-imidazol-1-yl]-1-piperidinyl]carbonyo-D-phenylalanyl]-4-(exo-8-methyl-8-aza-bicycto[3,2,1]oct-3-yl)-piperazin,
(AS) 1-[3,5-Dibrom-N-[[4-(1 ,3-dihydro-4-phenyl-2(2H)-oxoimidazol-1-yl)-1-piperidinyl]-carbonyl]-D-tyrosyl]-4-(1-piperidinyl)-piperidin,
(AT) 1-[4-Amino-3,5-dibrom-N-[[4-[1,3-dihydro-4-[3-(trifluormethyl)phenyl]-2(2H)-oxo-imidazol-1-yl]-1-piperidinyl]carbonyl]-D-phenylalanyl]-4-(1-ethyl-4-piperidinyl)-piperazin,
(AU) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(hexahydro-4-methyl-1 H-1,4-diazepin-1-yl)piperidin,
(AV) 1-[3,5-Dibrom-N-[[4-(3,4-dihydro-2(1H)-oxochinazolin-3-yl)-1-piperidinyl]-carbonyl]-D-tyrosyl]-4-[1-(methylsulfonyl)-4-piperidinyl]-piperidin,
(AW), 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidin,
(AX) 1-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-4-(hexahydro-1H-1-azepinyl)-piperidin,
(AY) 1-[3,5-Dibrom-N-[[4-(3,4-dihydro-2(1H)-oxochinazolin-3-yl)-1-piperidinyl]-carbonyl]-D-tyrosyl]-4-(1-methyl-4-piperidinyl)-piperidin,
(AZ) 1-[4-Amino-3,5-dibrom-N-[[4-(3,4-dihydro-2(1H)-oxochinazolin-3-yl)-1-piperidinyl]-carbonyl]-D-phenylalanyl]-4-(exo-8-methyl-8-azabicyclo[3,2,1]oct-3-yl)-piperazin,
(BA) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazin,
(BB) 1-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-4-(1-piperidinyl)-piperidin,
(BC) 1-[N⁶-Acetyl-N²-[3,5-dibrom-N-[[4-(3,4-dihydro-2(1H)-oxochinazolin-3-yl)-1-piperidinyl]carbonyl]-D-tyrosyl]-L-lysyl]-4-(4-pyridinyl)-perazin,
(BD) 1-[3,5-Dibrom-N-[[4-(1,3-dihydro-4.-phenyl-2(2H)-oxoimidazol-1-yl)-1-piperidinyl]-carbonyl]-D-tyrosyl]-4-(hexahydro-1H-1-azepinyl)-piperidin,
(BE) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidin,
(BF) 1-[4-Amino-3,5-dibrom-N-[[4-[1,3-dihydro-4-[3-(trifluorrrethyl)phenyl]-2(2H)-oxoimidazol-1-yl]-1-piperidinyl]carbonyl]-D-phenylalanyl]-4-(1-methyl-4-piperidinyl)-piperidin,
(BG) 1-[3,5-Dibrom-N-[[4-(3,4-dihydro-2(1H)-oxochinazolin-3-yl)-1-piperidinyl]-carbonyl]-D-tyrosyl]-4-[1-(hydroxycarbonylmethyl)-4-piperidinyl]-piperidin,
(BH) 1-[4-Amino-3,5-dibrom-N-[[4-[3,4-dihydro-2(1H)-oxochinazolin-3-yl]-1-piperidinyl]carbonyl]-D-phenylalanyl]-4-(1-methylsulfonyl-4-piperidinyl)-piperidin,
(BI) 1-[3,5-Dibrom-N-[[4-(3,4-dihydro-2(1H)-oxochinazolin-3-yl)-1-piperidinyl]-carbonyl]-D-tyrosyl]-4-(4-piperidinyl)-piperidin,
(BJ) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidin,
(BK) 1-[4-Amino-3,5-dibrom-N-[[4-(1,3-dihydro-4-(3-hydroxyphenyl)-2(2H)-oxo-imidazol-1-yl)-1-piperidinyl]carbonyl]-D-phenylalanyl]-4-(1-methyl-4-piperidinyl)-piperidin,
(BL) 1-[3,5-Dibrom-N-[[4-(3,4-dihydro-2(1H)-oxochinazolin-3-yl)-1-piperidinyl]-carbonyl]-D-tyrosyl]-4-(hexahydro-1 H-1-azepinyl)-piperidin,
(BM) 1-[4-Amino-3,5-dibrom-N-[[4-(1,3-dihydro-4-phenyl-2(-2H)-oxoimidazol-1-yl)-1-piperidinyl]carbonyl]-D-phenylalanyl]-4-(1-piperidinyl)-piperidin,
(BN) 1-[4-Amino-3,5-dibrom-N-[[4-[4-(3-bromphenyl)-1,3-dihydro-2(2H)-oxoimidazol-1-yl]-1-piperidinyl]carbonyl]-D-phenylalanyl]-4-(exo-8-methyl-8-azabicyclo[3,2,1]oct-3-yl)-piperazin,
(BO) 1-[4-Amino-3,5-Dibrom-N-[[4-(3,4-dihydro-2(1 H)-oxochinazolin-3-yl)-1-piperidinyl]carbonyl]-D-phenylalanyl]-4-(1-ethyl-4-piperidinyl)-piperidin,
(BP) 1-[4-Amino-3,5-Dibrom-N-[[4-(3,4-dihydro-2(1 H)-oxochinazolin-3-yl)-1-piperidinyl]carbonyl]-D-phenylalanyl]-4-(1-ethyl-4-piperidinyl)-piperazin ,
(BQ) 1-[4-Amino-3,5-dibrom-N-[[4-[1,3-dihydro-4-(3-methoxyphenyl)-2(2H)-oxo-imidazol-1-yl]-1-piperidinyl]carbonyl]-D-phenylalanyl]-4-(exo-8-methyl-8-azabicyclo[3,2,1]oct-3-yl)-piperazin,
(BR) 1-[3,5-Dibrom-N-[[4-(3,4-dihydro-2(1H)-oxochinazolin-3-yl)-1-piperidinyl]-carbonyl]-D-tyrosyl]-4-[1-(cyclopropylmethyl)-4-piperidinyl]-piperidin,
(BS) 1-[4-Amino-3,5-Dibrom-N-[[4-(3,4-dihydro-2(1H)-oxochinazolin-3-yl)-1-piperidinyl]carbonyl]-D-phenylalanyl]-4-(hexahydro-1H-1-azepinyl)-piperidin,
(BT) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin,
(BU) 1-[3,5-Dibrom-N-[[4-(1,3-dihydro-4-phenyl-2(2H)-oxoimidazol-1-yl)-1-piperidinyl]carbonyl]-D-tyrosyl]-4-(4-pyridinyl)-piperidin,
(BV) 1-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-4-(1-methyl-4-piperidinyl)-piperazin,
(BW) 1-[N2-[3,5-Dibrom-N-[[4-[1,3-dihydro-4-[3-(trifluormethyl)phenyl]-2(2H)-oxo-imidazol-1-yl]-1-piperidinyl]carbopyl]-D-tyrosyl]-L-lysyl]-4-(4-pyridinyl)-piperazin,
(BX) 1-[3,5-Dibrom-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidin,
(BY) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
(BZ) 1-[4-Amino-3,5-dibrom-N-[[4-[1,3-dihydro-4-[3-(trifluormethyl)phenyl]-2(2H)-oxo-imidazol-1-yl]-1-piperidinyl]carbonyl]-D-phenylalanyl]-4-(hexahydro-1H-1-azepinyl)-piperidin,
(CA) 1-[4-Amino-3,5-dibrom-N-[[4-[1,3-dihydro-4-[3-(trifluormethyl)phenyl]-2(2H)-oxo-imidazol-1-yl]-1-piperidinyl]carbonyl]-D-phenylalanyl]-4.-(1-methyl-4-piperidinyl)-piperazin,
(CB) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(hexahydro-1H-1-azepinyl)-piperidin,
(CC) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(4-pyridinyl)-piperazin,
(CD) 1-[3,5-Dibrom-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidin,
(CE) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazin,
(CF) 1-[3,5-Dibrom-N-[[4-[3,4-dihydro-2(1H)-oxochinazolin-3-yl]-1-piperidinyl]-carbonyl]-D-tyrosyl]-4-(1-methyl-4-piperidinyl)-piperazin,
(CG) 1-[4-Amino-3,5-dibrom-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidin,
(CH) 1-[4-Amino-3,5-dibrom-N-[[4-[3,4-dihydro-2(1H)-oxochinazolin-3-yl]-1-piperidinyl]carbonyl]-D-phenylalanyl]-4-(1-pyrrolidinyl)-piperidin,
(CI) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(hexahydro-1H-1-azepinyl)-piperidin und
(CJ) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazin,
deren Tautomere, deren Diastereomere, deren Enantiomere und deren Gemische.

### Stand der Technik

Die Verbindungen der voranstehend genannten Gruppe (A) bis (CJ) stellen hochwirksame CGRP-Antagonisten zur Behandlung von Migräne dar, deren Applikation mittels klassischer Darreichungsformen nicht auf oralem Wege möglich ist, da die Substanz sowohl als Wirkstoffbase als auch in Form ihrer Salze oral nur eine geringe Bioverfügbarkeit aufweist.

Die orale Applikation eines Wirkstoffs stellt für den Patienten generell die praktikabelste Einnahmeform dar. Da akute Migräneanfälle häufig mit Übelkeit und Erbrechen einhergehen, kann die orale Aufnahme eines Antimigränemittels jedoch erschwert oder sogar unmöglich sein.
Bei einem akuten Migräneanfall ist es unerläßlich, dass die Wirkung des verabreichten Medikamentes sofort oder zumindest sehr bald nach der Einnahme einsetzt, so dass das Erreichen wirksamer Plasmaspiegel kurz nach der Applikation von Vorteil ist. Die Möglichkeiten in diesem Zusammenhang wären die intravenöse, intramuskuläre oder subkutane Gabe durch Injektionen. Diese komplexe Vorgehensweise, zumal mit einem erhöhten Infektionsrisiko verbunden, ist jedoch zur allgemeinen Anwendbarkeit nicht geeignet.

Als Alternative zur Resorption über den Gastrointestinaltrakt bietet die Nasenschleimhaut eine für die Resorption von Arzneistoffen prinzipiell ebenfalls geeignete Oberfläche. Im Nasenepithel ist die Biotransformation von Arzneistoffen wesentlich geringer ausgeprägt als im Gastrointestinaltrakt oder in der Leber, so dass mit einer schnellen Absorption geeigneter Wirkstoffe in den Blutkreislauf und dem Fehlen eines first-pass-Metabolismus zu rechnen ist. Unter diesen Voraussetzungen müßte die schnelle Erzielung hoher Plasmaspiegel ähnlich dem, der durch Injektionen erreicht wird, möglich sein.

Übliche nasale Anwendungen benötigen Treibmittel, um eine entsprechende Applikation zu gewährleisten. In der Regel sind handelsübliche Treibmittel Vertreter der Fluor-Chlor-Kohlenwasserstoffe sowie der Fluorkohlenwasserstöffe, so dass entsprechende Formulierungen im Hinblick auf den Umweltschutz von Nachteil sind.
Wäßrige Formulierungen in Pump- oder Ventilsprays, die ohne Treibmittel anwendbar sind, müssen durch Zusatz von Konservierungsstoffen haltbar gemacht werden. Konservierungsstoffe sind jedoch häufig mit einem hohen Allergiepotential verbunden, weshalb Allergiker diese Arzneimittel nicht verwenden können. Zudem sind alle zugelassenen Konservierungsmittel cytotoxisch und beeinträchtigen die Ziliarfunktion und dadurch auch die Clearance.
Ein weiterer Nachteil der nasalen Resorption von Wirkstoffen aus wäßrigen Lösungen ist deren pH-Wert-Abhängigkeit. Das optimale Milieu für die Zilien der Nasenschleimhaut liegt bei einem pH-Wert von 7 bis 9. Die maximale Resorption wird jedoch bei einem pH-Wert unterhalb von 6 erreicht.

### Problemstellung

Die Aufgabe der vorliegenden Erfindung bestand in der Bereitstellung einer schnell bioverfügbaren Formulierung für die eingangs erwähnten hochwirksamen CGRP-Antagonisten (A) bis (CJ), mit der das Problem der geringen oralen Verfügbarkeit umgangen wird. Die erfindungsgemäße Formulierung sollte zur Behandlung der akuten, bei Migräne sehr plötzlich eintretenden Schmerzzustände einen schnellen Wirkungseintritt zeigen. Das bedeutet, dass eine rasche Aufnahme des Wirkstoffs und ein schneller Anstieg des Plasmaspiegels gewährleistet sein müssen. Dabei sollte die erfindungsgemäße Formulierung in einer Anwendungsform zur Verfügung stehen, welche auch die Verwendung bei nur sporadisch auftretenden Migräne-Attacken berücksichtigt. Weiterhin sollte die Anfälligkeit des wirksamen Agens für oxidative Zersetzung während der gesamten Produkt-Lagerdauer im Sinne eines effektiven Sauerstoffschutzes berücksichtigt werden.

### Beschreibung der Erfindung

Ein hoher Plasmaspiegel der Wirkstoffe (A) bis (CJ) und damit ein schneller Wirkungseintritt zur Behandlung von akuten Schmerzzuständen innerhalb kürzester Zeit läßt sich neben der intravenösen Gabe erfindungsgemäß über die Nase als Aufnahmeorgan realisieren.

Im Rahmen der vorliegenden Erfindung wurde nun überraschend gefunden, dass die eingangs erwähnten CGRP-Antagonisten (A) bis (CJ) in Form ihrer Salze mit physiologisch verträglichen solubilisierenden Säuren, bevorzugt mit Salzsäure in Form der Hydrochloride, durch eine Darreichung über die Nase in ausreichendem Maße bioverfügbar gemacht werden können.

Ein erster erfindungsgemäßer Gegenstand ist somit eine pharmazeutische Zusammensetzung für die nasale Applikation in Form einer wässrigen Lösung, umfassend
(a) 2% bis 25 % g/v, vorzugsweise 10% bis 20% g/v eines Wirkstoffs, ausgewählt aus der Gruppe (A) bis (CJ) bestehend aus
   (A) 1 -[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,
   (B) 1-[4-Amino-3,5-dibrom-N-[[4-(2,3,4,5-tetrahydro-2(1 H)-oxo-1,3-benzodiazepin-3-yl)-1-piperidinyl]carbonyl]-D-phenylalanyl]-4-(1-piperidinyl)-piperidin,
   (C) 1-[N²-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperazin,
   (D) 1-[N²-[4-Amino-3,5-dibrom-N-[[4-(3,4-dihydro-2(1 H)-oxochinazolin-3-yl)-1-piperidinyl]carbonyl]-D-phenylalanyl]-L-lysyl]-4-(4-pyridinyl)-piperidin,
   (E) 1-[N²-[3,5-Dibrom-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazin,
   (F) 1-[N²-[4-Amino-3,5-dibrom-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazin,
   (G) 1-[3,5-Dibrom-N-[[4-(3,4-dihydro-2(1H)-oxothieno[3,4-d]pyrimidin-3-yl)-1-piperidinyl]carbonyl]-D-tyrosyl]-4-(1-piperidinyl)-piperidin,
   (H) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidin,
   (I) 1-[4-Amino-3,5-dibrom-N-[[4-(2,4-dihydro-5-phenyl-3(3H)-oxo-1,2,4-triazol-2-y1)-1-piperidinyl]carbonyl]-D-phenylalanyl]-4-(9-piperidinyl)-piperidin,
   (J) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,
   (K) 1-[4-Amino-3,5-dibrom-N-[[4-(3,4-dihydro-2(1H)-oxothieno[3,2-d]pyrimidin-3-yl)-1-piperidinyl]carbonyl]-D-phenylalanyl]-4-(1-piperidinyl)-piperidin,
   (L) 1-[4-Amino-3,5-dibrom-N-[[4-[1,3-dihydro-4-[3-(tri-fluoromethyl)phenyl]-2(2H)-oxoimidazol-1-yl]-1-piperidin-yl]carbonyl]-D-phenylalanyl]-4-(1-ethyl-4-piperidinyl)-piperidin,
   (M) 1-[4-Amino-3,5-dibrom-N-[[4-[3,4-dihydro-2(1H)-oxochinazolin-3-yl]-1-piperidinyl]carbonyl]-D-phenylalanyl]-4-(1-hexyl-4-piperidinyl)-piperidin,
   (N) 1-[4-Amino-3,5-dibrom-N-[[4-[3,4-dihydro-2(1H)-oxochinazolin-3-yl]-1- piperidinyl]carbonyl]-D-phenylalanyl]-4-(1-cyclopropylmethyl-4-piperidinyl)-piperidin,
   (O) 1-[N-[[4-[3,4-Dihydro-2(1H)-oxochinazolin-3-yl]-1-piperidinyl]carbonyl]-3-ethenyl-D,L-phenylalanyl]-4-(hexahydro-1 H-1-azepinyl)-piperidin,
   (P) (R,S)-1-[4-[4-(3,4-Dihydro-2(1 H)-oxochinazolin-3-yl)-1-piperidinyl]-2-[(4-hydroxy-3,5-dimethylphenyl)methyl]-1,4-dioxobutyl]-4-(1-piperidinyl)-piperidin,
   (Q) 1-[4-Amino-3,5-dibrom-N-[[4-[N-(aminocarbonyl)-N-phenylamino]-1-piperidinyl]carbonyl]-D-phenylalanyl]-4-(1-piperidinyl)-piperidin,
   (R) 1-[4-Amino-3,5-dibrom-N-[[4-(3,4-dihydro-2(1H)-oxochinazolin-3-y1)-1-piperidinyl]carbonyl]-D-phenylalanyl]-4-(5-methoxy-4-pyrimidinyl)-piperazin,
   (S) 1-[4-Amino-3,5-dibrom-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidin,
   (T) 1-[4-Amino-3,5-dibrom-N-[[4-2(1H)-oxochinolin-3-yl]-1-piperidinyl]carbonyl]-D-phenylalanyl]-4-(1-piperidinyl)-piperidin,
   (U) 1-[4-Amino-3,5-dibrom-N-[[4-[3,4-dihydro-2(1H)-oxochinazolin-3-yl]-1-piperidinyl]carbonyl]-D-phenylalanyl]-4-[3-(dimethylamino)propyl]-piperazin,
   (V) 1-[4-Amino-3,5-dibrom-N-[[4-[3,4-dihydro-2(1H)-oxochinazolin-3-yl]-1-piperidinyl]carbonyl]-D-phenylalanyl]-4-(4-methyl-1-piperazinyl)-piperidin,
   (W) 1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperidinyl)carbonyl]-piperazin,
   (X) 1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperazinyl)carbonyl]-piperazin,
   (Y) 1-[4-Amino-3,5-dibrom-N-[[4-(3,4-dihydro-2(1H)-oxochinazolin-3-yl)-1-piperidinyl]carbonyl]-D-phenylalanyl]-4-[4-[4-(dimethylamino)butyl]phenyl]-piperazin,
   (Z) 1-[4-Amino-3,5-dibrom-N-[[4-(3,4-dihydro-2(1H)-oxochinazolin-3-yl)-1-piperidinyl]carbonyl]-D-phenylalanyl]-4-[4-(dimethylamino)-1-piperidinyl]-piperidin,
   (AA) 1-[N²-[[4-(1,3-Dihydro-4-phenyl-2(2H)-oxoimidazol-1-yl)-1-piperidinyl]carbonyl]-N'-methyl-D-tryptyl]-4-(4-methyl-1-piperazinyl)-piperidin,
   (AB) 1-[N²-[[4-(1,3-Dihydro-4-phenyl-2(2H)-oxoimidazol-1-yl)-1-piperidinyl]carbonyl]-N'-(1,1-dimethylethoxycarbonyl)-D-tryptyl]-4-(1-methyl-4-piperidinyl)-piperidin,
   (AC) (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,
   (AD) (R,S)-1-[4-[4-(3,4-Dihydro-2(1H)-oxochinazolin-3-yl)-1-piperidinyl]-2-[(3,5-dibrom-4-methoxyphenyl)methyl]-1,4-dioxobutyl]-4-(1-methyl-4-piperidinyl)-piperidin,
   (AE) (R,S)-1-[4-[4-(3,4-Dihydro-2(1H)-oxochinazolin-3-yl)-1-piperidinyl]-2-[(3,4-dibromphenyl)methyl]-1,4-dioxobutyl]-4-(4-methyl-1-piperazinyl)-piperidin,
   (AF) 1-[N²-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin,
   (AG) 1-[4-Amino-3,5-dibrom-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidin,
   (AH) 1-[N²-[4-Amino-3,5-dibrom-N-[[4-(1,3-dihydro-2(2H)-oxobenzimidazol-1-yl)-1-piperidinyl]carbonyl]-D-phenylalanyl]-N⁶,N⁶-dimethyl-L-lysyl]-4-(4-pyridinyl)-piperazin,
   (AI) 1-[N²-[4-Amino-3,5-dibrom-N-[[4-(3,4-dihydro-2(1H)-oxochinazolin-3-yl)-1-piperidinyl]carbonyl]-D-phenylalanyf]-N⁶,N⁶-dimethyl-L-lysyl]-4-(4-pyridinyl)-piperazin,
   (AJ) (R,S)-1-[2-(4-Amino-3,5-dibrombenzoyl)-4-[4-(3,4-dihydro-2(1H)-oxochinazolin-3-yl)-1-piperidinyl]-4-oxobutyl]-4-(1-piperidinyl)-piperidin,
   (AK) 1-[4-Amino-3,5-dibrom-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidin,
   (AL) 1-[4-Amino-3,5-dibrom-N-[[4-[1,3-dihydro-2(2H)-oxoimidazo[4,5-c]chinolin-3-yl]-1-piperidinyl]carbonyl]-D-phenylalanyl]-4-(1-piperidinyl)carbonyl]-piperidin,
   (AM) 1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,
   (AN) 1-[N²-[3,5-Dibrom-N-[[4-(3,4-dihydro-2(1H)-oxochinazolin-3-yl)-1-piperidinyl]carbonyl]-D-tyrosyl]-N⁶,N⁶-dimethyl-L-lysyl]-4-(4-pyridinyl)-piperazin,
   (AO) 1-[4-Amino-N-[[4-[4-(3-bromphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
   (AP) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidin,
   (AQ) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-piperidinyl)-piperidin,
   (AR) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(exo-8-methyl-8-azabicyclo[3,2,1]oct-3-yl)-piperazin,
   (AS) 1-[3,5-Dibrom-N-[[4-(1,3-dihydro-4-phenyl-2(2H)-oxoimidazol-1-yl)-1-piperidinyl]carbonyl]-D-tyrosyl]-4-(1-piperidinyl)-piperidin,
   (AT) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-ethyl-4-piperidinyl)-piperazin,
   (AU) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(hexahydro-4-methyl-1H-1,4-diazepin-1-yl)piperidin,
   (AV) 1-[3,5-Dibrom-N-[[4-(3,4-dihydro-2(1H)-oxochinazolin-3-yl)-1-piperidinyl]-carbonyl]-D-tyrosyl]-4-[1-(methylsulfonyl)-4-piperidinyl]-piperidin,
   (AW) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidin,
   (AX) 1-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-4-(hexahydro-1 H-1 - azepinyl)-piperidin,
   (AY) 1-[3,5-Dibrom-N-[[4-(3,4-dihydro-2(1 H)-oxochinazolin-3-yl)-1-piperidinyl]-carbonyl]-D-tyrosyl]-4-(1-methyl-4-piperidinyl)-piperidin,
   (AZ) 1-[4-Amino-3,5-dibrom-N-[[4-(3,4-dihydro-2(1H)-oxochinazolin-3-yl)-1-piperidinyl]carbonyl]-D-phenylalanyl]-4-(exo-8-methyl-8-azabicyclo[3,2,1]oct-3-yl)-piperazin,
   (BA) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazin,
   (BB) 1-[3,5-Dibrom-N-[[4-[1,3-dihydro-4-[3-(trifluorrrethyl)phenyl]-2(2H)-oxoimidazol-1-yl]-1-piperidinyi]carbonyl]-D-tyrosyl]-4-(1 -piperidinyl piperidin,
   (BC) 1-[N⁶-Acetyl-N²-[3,5-dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,
   (BD) 1 -[3,5-Dibrom-N-[[4-(1,3-dihydro-4-phenyl-2(2H)-oxoimidazol-1-yl)-1-piperidinyl]carbonyl]-D-tyrosyl]-4-(hexahydro-1 H-1-azepinyl)-piperidin,
   (BE) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidin,
   (BF) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperidin,
   (BG) 1-[3,5-Dibrom-N-[[4-(3,4-dihydro-2(1H)-oxochinazolin-3-yl)-1-piperidinyl]-carbonyl]-D-tyrosyl]-4-[1-(hydroxycarbonylmethyl)-4-piperidinyl]-piperidin,
   (BH) 1-[4-Amino-3,5-dibrom-N-[[4-[3,4-dihydro-2(1 H)-oxochinazolin-3-yl]-1-piperidinyl]carbonyl]-D-phenylalanyl]-4-(1-methylsulfonyl-4-piperidinyl)-piperidin,
   (BI) 1-[3,5-Dibrom-N-[[4-(3,4-dihydro-2(1H)-oxochinazolin-3-yl)-1-piperidinyl]-carbonyl]-D-tyrosyl]-4-(4-piperidinyl)-piperidin,
   (BJ) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidin,
   (BK) 1-[4-Amino-3,5-dibrom-N-[[4-(1,3-dihydro-4-(3-hydroxyphenyl)-2(2H)-oxo-imidazol-1-yl)-1-piperidinyl]carbonyl]-D-phenylalanyl]-4-(1-methyl-4-piperidinyl)-piperidin,
   (BL) 1-[3,5-Dibrom-N-[[4-(3,4-dihydro-2(1H)-oxochinazolin-3-yl)-1-piperidinyl]-carbonyl]-D-tyrosyl]-4-(hexahydro-1H-1-azepinyl)-piperidin,
   (BM) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-piperidinyl)-piperidin,
   (BN) 1-[4-Amino-3,5-dibrom-N-[[4-[4-(3-bromphenyl)-1,3-dihydro-2(2H)-oxoimidazol-1-yl]-1-piperidinyl]carbonyl]-D-phenylalanyl]-4-(exo-8-methyl-8-azabicyclo[3,2,1]oct-3-yl)-piperazin,
   (BO) 1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperidin,
   (BP) 1-[4-Amino-3,5-Dibrom-N-[[4-(3,4-dihydro-2(1 H)-oxochinazolin-3-yl)-1-piperidinyl]carbonyl]-D-phenylalanyl]-4.-(1-ethyl-4-piperidinyl)-piperazin,
   (BQ) 1-[4-Amino-3,5-dibrom-N-[[4-[1,3-dihydro-4-(3-methoxyphenyl)-2(2H)-oxo-imidazol-1-yl]-1-piperidinyl]carbonyl]-D-phenylalanyl]-4-(exo-8-methyl-8-azabicyclo[3,2,1]oct-3-yl)piperazin,
   (BR) 1-[3,5-Dibrom-N-[[4-(3,4-dihydro-2(1H)-oxochinazolin-3-yl)-1-piperidinyl]-carbonyl]-D-tyrosyl]-4-[1-(cyclopropylmethyl)-4-piperidinyl]-piperidin,
   (BS) 1-[4-Amino-3,5-Dibrom-N-[[4-(3,4-dihydro-2(1H)-oxochinazolin-3-yl)-1-piperidinyl]carbonyl]-D-phenylalanyl]-4-(hexahydro-1H-1-azepinyl)-piperidin,
   (BT) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin,
   (BU) 1-[3,5-Dibrom-N-[[4-(1,3-dihydro-4-phenyl-2(2H)-oxoimidazol-1-yl)-1-piperidinyl]carbonyl]-D-tyrosyl]-4-(4-pyridinyl)-piperidin,
   (BV) 1-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-4-(1-methyl-4-piperidinyl)-piperazin,
   (BW) 1-[N2-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-L-lysyl]-4-(4-pyridinyl)-piperazin,
   (BX) 1-[3,5-Dibrom-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidin,
   (BY) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
   (BZ) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(hexahydro-1 H-1-azepinyl)-piperidin,
   (CA) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperazin,
   (CB) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(hexahydro-1H-1-azepinyl)-piperidin,
   (CC) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(4-pyridinyl)-piperazin,
   (CD) 1-[3,5-Dibrom-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidin,
   (CE) 1-[4-Amino-3,5-dibrom-N-[[4-[1,3-dihydro-4-phenyl-2(2H)-oxoimidazol-1 - yl]-1-piperidinyl]carbonyl]-D-phenylalanyl]-4-[4-(1-oxoethyl)phenyl]-piperazin,
   (CF) 1-[3,5-Dibrom-N-[[4-[3,4-dihydro-2(1H)-oxochinazolin-3-yl]-1-piperidinyl]-carbonyl]-D-tyrosyl]-4-(1-methyl-4-piperidinyl)-piperazin,
   (CG) 1-[4-Amino-3,5-dibrom-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidin,
   (CH) 1-[4-Amino-3,5-dibrom-N-[[4-[3,4-dihydro-2(1H)-oxochinazolin-3-yl]-1-piperidinyl]carbonyl]-D-phenylalanyl]-4-(1-pyrrolidinyl)-piperidin,
   (CI) 1-[4-Amino-3,5-dibrom-N-[[4-(1,3-dihydro-4-phenyl2(2H)-oxoimidazol-1-yl)-1-piperidinyl]carbonyl]-D-phenylalanyl]-4-(hexahydro-1 H-1-azepinyl)-piperidin und
   (CJ) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazin, und
(b) 1.2 bis 2 Äquivalente einer physiologisch verträglichen, solubilisierenden Säure, wobei der Wirkstoff mittels der Säure in das entsprechende Salz übergeführt wird und in anionischer Form gelöst vorliegt.

In einer bevorzugten erfindungsgemäßen Ausführungsform sind die Verbindungen
(A) 1-[N²-[3,5-Dibrom-N-[[4-(3,4-dihydro-2(1 H)-oxochinazolin-3-yl)-1-piperidinyl]-carbonyl]-D-tyrosyl]-L-lysyl]-4-(4-pyridinyl)-piperazin und
(B) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,
enthalten.

Zur Solubilisierung der nur wenig wasserlöslichen Wirkstoffbasen werden diese mit 1.2 bis 2 Äquivalenten, besonders bevorzugt mit 1.6 bis 1.9 Äquivalenten, einer Säure *in situ* zu dem entsprechenden Säureadditionssalz umgesetzt. In einer ganz besonders bevorzugten erfindungsgemäßen Ausführungsform wird der Wirkstoff mit 1.75 Äquivalenten einer Säure umgesetzt.
Auf diese Weise lassen sich selbst hochkonzentrierte Lösungen realisieren, deren Anwendbarkeit im Sinne einer nasalen Gabe jedoch dadurch Grenzen gesetzt sind, dass die mit der Konzentration zunehmende Viskosität ein Versprühen als feiner Sprühnebel mit einer geeigneten Sprühgeometrie (Kegel = "plume") bzw. mit einer geeigneten Teilchengröße erschwert bis unmöglich macht. Geeignete anorganische und organische physiologisch verträgliche Säuren sind beispielsweise Salzsäure, Phosphorsäure, Methansulfonsäure, Essigsäure, Ameisensäure oder Bernsteinsäure.

Überraschenderweise wurde gefunden, dass das molare Verhältnis von Wirkstoffbase zur solubilisierenden Säure die Mischbarkeit der schwach sauren Wirkstofflösung mit physiologischen Körperflüssigkeiten wie Plasma oder Nasen-Mucus beeinflusst, dergestalt, dass die Wirksubstanz im physiologischen, neutralen pH-Bereich länger bzw. in höherer Konzentration in Lösung bleibt je höher der molare Überschuss an solubilisierender Säure ist. Eine längere Verweilzeit des Wirkstoffs in gelöster Form auf der Nasenschleimhaut sollte demnach zu einer höheren Resorptionsrate und höheren systemischen Verfügbarkeit führen.

Eine weitere erfindungsgemäße pharmazeutische Zusammensetzung kann daher auch ein physiologisch verträgliches Salz der vorstehend genannten Wirkstoffe der Gruppe (A) bis (CJ) in einer wässrigen Lösung der voranstehend genannten Konzentrationen enthalten, der 0.2 bis 1 Äquivalente, bevorzugt 0.6 bis 0.9 Äquivalente, der entsprechenden Säure zugesetzt werden.
Eine besonders bevorzugte Ausführung enthält Salzsäure als solubilisierende Säure in einem Überschuss von 0.75 Moläquivalenten bezogen auf die eingesetzte Wirkstoffbase.

Eine weitere erfindungsgemäße Ausführung einer pharmazeutischen Zusammensetzung für die nasale Applikation enthält zusätzlich einen oder mehrere "absorption enhancer" wie z.B. Caprylocaproyl-macrogolglycerid (Labrasol ^{™}) oder Lysolecithin in einer Konzentration von 0.1% bis 5% g/v, vorzugsweise im Bereich 0.5% bis 3% g/v. Diese absorption enhancer erhöhen die Permeabilität der Mucosa, woraus letztlich eine höhere systemische Verfügbarkeit des Wirkstoffs resultiert.

Eine weitere erfindungsgemäße Ausführung einer pharmazeutischen Zusammensetzung für die nasale Applikation enthält zusätzlich ein gelbildendes Agens wie z.B. Hydroxypropylmethyl-cellulose, Polymere der Acrylsäure (z.B. Carbopol 934) oder Xanthan in einer Konzentration von 0.05% bis 1 % g/v, vorzugsweise im Bereich 0.1 % bis 0.5% g/v. Diese erhöhen die Viskosität und damit die Verweilzeit der nasal applizierten Zubereitung durch eine Modulation des Clearing Mechanismus' der Nasenschleimhaut.

Eine weitere Ausführungsform der nasalen Zubereitung enthält zusätzlich ein liposomenbildendes Phospholipid, z.B. Sojalecithin S 100, in einer Konzentration von 2% bis 10% g/v, vorzugsweise im Bereich von 3% bis 8% g/v, gegebenenfalls zusätzlich enthaltend eine geringe Menge eines Phospholipid-Hydrolyseprodukts; z.B. Lysolecithin, in einer Konzentration von 0.5% bis 1 % g/v. Die Liposomen-basierte Formulierung zeigt eine höhere Permeationsrate durch die Mucosa und damit eine höhere systemische Verfügbarkeit des Wirkstoffs.

Weiterhin können den vorstehend genannten erfindungsgemäßen Ausführungsformen optional ein oder mehrere Isotonantien zugesetzt werden, beispielsweise Natriumchlorid in Konzentrationen von 0.3% bis 3% g/v, Mannitol in Konzentrationen von 1.5% bis 15% g/v, Lactose in Konzentrationen von 3% bis 20% g/v oder Xylitol in Konzentrationen von 1.5% bis 15% g/v. Insofern ist ein weiterer Teilaspekt der vorliegenden Erfindung die Identifizierung einer Sekundärverpackung, die diesen Langzeit-Schutz gewährleistet. Geeignet sind Beutel- bzw. Schlauchbeutelverpackungen aus Aluminium bzw. metallisierten Folien, wenn diese unter Schutzgasatmosphäre mit dem Primärpackmittel (z.B. UDS) befüllt und verschweißt werden. Eine weitere Alternative stellt die Verpackung in Transparent-Folien dar, sofern durch Mitverpackung von Sauerstoff-Fängern/"oxygen absorbers" (z.B. solche, die pulverisiertes elementares Eisen oder Eisenoxid beinhalten, das den Sauerstoff unter Bildung von Eisenoxid in verschiedenen Oxidationsstufen des Eisens absorbiert) auch mit Transparent-Folien ein ausreichender Langzeitschutz realisiert werden kann.

Ein zweiter erfindungsgemäßer Gegenstand ist ein Verfahren zur Herstellung einer der voranstehend genannten pharmazeutischen Zusammensetzungen, umfassend die Schritte
(a) Auflösen von 2 bis 25% g/v, bezogen auf die pharmazeutische Zusammensetzung, eines Wirkstoffs ausgewählt aus der Gruppe (A) bis (CJ) gemäß Anspruch 1 in einer wässrigen Lösung, umfassend 1.2 bis 2 Äquivalente einer physiologisch verträglichen, solubilisierenden Säure oder
(b) Auflösen eines Salzes eines Wirkstoffs ausgewählt aus der Gruppe (A) bis (CJ) gemäß Anspruch 1, gebildet mit einer physiologisch verträglichen, solubilisierenden Säure, in Wasser in einer Menge, so dass der Wirkstoffgehalt der Zusammensetzung bezogen auf den Wirkstoff 2% bis 25% g/v beträgt, und
(c) Zusatz von 1.2 bis 2 Äquivalenten physiologisch verträglicher, solubilisierender Säure und
(d) optional Zusatz eines oder mehrerer Hilfsstoffe, ausgewählt aus absorption enhancern, gelbildenden Agentien und liposomenbildenden Phospholipiden sowie
(e) gegebenenfalls Verpackung der so erhaltenen Lösung unter Schutzgas als Einzeldosen in Primärpackmitteln, wobei die Primärpackmittel in eine Sekundärverpackung, ausgeführt als Beutel- oder Schlauchbeutelverpackung aus Aluminium, metallisierter Folie oder Transparent-Folie, unter Schutzgasatmosphäre, gegebenenfalls zusammen mit einem Sauerstoff-Fänger, eingebracht sind.

### Experimentelle Daten

### (1) Stabilität

Stabilitätsprüfung höher konzentrierter erfindungsgemäßer Nasalspray-Lösungen:

Die nachfolgende Tabelle zeigt, dass die oxidative Zersetzung des nachstehend aufgeführten Wirkstoffs

### (A) 1-[N²-[3,5-Dibrom-N-[[4-(3,4-dihydro-2(1H)-oxochinazolin-3-yl)-1-piperidinyl]-carbonyl]-D-tyrosyl]-L-lysyl]-4-(4-pyridinyl)-piperazin,

in Form des Hydrochlorids in wäßriger Lösung über einen langen Zeitraum praktisch unverändert ist bzw. nur wenig zunimmt. Die zum Teil (insbesondere bei 40°C) hohe Gesamtzersetzung beruht auf Hydrolyse.

Der in der Tabelle 1 angegebene Gehalt an Wirkstoff (A) bezieht sich jeweils auf die ursprünglich eingewogene freie Base.

**Tabelle 1:**

| Wirkstoff-Konzentration | | | **15 %** | **20 %** | **30 %** |
|---|---|---|---|---|---|
| **Lagerzeit** | **Lager-temp:** | **Prüfpunkte** | | | |
| **Anfangswert** | | Gehalt an Wirkstoff (A) | 148,6mg/ml | 197,9mg/ml | 269mg/ml |
| | | Zersetzung durch Oxidation | 0,59% | 0,56% | 0,56% |
| | | Gesamt-Zersetzung | 1,23% | 1,27% | 1,20% |
| | | Sauerstoffgehalt im Alu-Beutel | 0,3% | 0,3% | 0,9% |
| **6-Wochen-Wert** | 25°C | Gehalt an Wirkstoff (A) | 148,1mg/ml | 198,7mg/ml | 264mg/ml |
| | | Zersetzung durch Oxidation | 0,72% | 0,54% | 0,62% |
| | | Gesamt-Zersetzung | 1,60% | 1,09% | 1,19% |
| | | Sauerstoffgehalt im Alu-Beutel | 0,3% | 0,2% | 0,4% |
| | 40°C | Gehalt an Wirkstoff (A) | 142,3mg/ml | 192,1mg/ml | 260mg/ml |
| | | Zersetzung durch Oxidation | 0,52% | 0,55% | 0,58% |
| | | Gesamt-Zersetzung | 4,46% | 3,97% | 3,95% |
| | | Sauerstoffgehalt im Alu-Beutel | 03% 0,3% | 06% 0,6% | 04% 0,4% |
| **3-Monats-Wert** | 25°C | Gehalt an Wirkstoff (A) | 147,7mg/ml | 202,8mg/ml | 270mg/ml |
| | | Zersetzung durch Oxidation | 0,64% | 0,53% | 0,60% |
| | | Gesamt-Zersetzung | 1,69% | 1,50% | 1,48% |
| | | Sauerstoffgehalt im Alu-Beutel | 0,4% | 0,3% | 0,3% |
| | 40°C | Gehalt an Wirkstoff (A) | 139,4mg/ml | 191,4mg/ml | 260mg/ml |
| | | Zersetzung durch Oxidation | 0,55% | 0,47% | 0,59% |
| | | Gesamt-Zersetzung | 8,22% | 8,23% | 7.81% |
| | | Sauerstoffgehalt im Alu-Beutel | 0,4% | 0 6% | 0,3% |
| **6-Monats-Wert** | 25°C | Gehalt an Wirkstoff (A) | 147,7mg/ml | 201,8m/ml | 273mg/ml |
| | | Zersetzung durch Oxidation | 0,82% | 0,77% | 0,91% |
| | | Gesamt-Zersetzung | 2,07% | 1,99% | 2,04% |
| | | Sauerstoffgehalt im Alu-Beutel | 0,7% | 0,4% | 0,4% |
| | 40°C | Gehalt an Wirkstoff (A) | 131,7mg/ml | 182,8mg/ml | 246mg/ml |
| | | Zersetzung durch Oxidation | 0,78% | 0,74% | 0,84% |
| | | Gesamt-Zersetzung | 15.33% | 14,61% | 13,36% |
| | | Sauerstoffgehalt im Alu-Beutel | 0,4% | 0,5% | 0,4% |

### (2) Pharmakokinetik

Die nachstehende Tabelle 2 zeigt die absolute Bioverfügbarkeit verschiedener erfindungsgemäßer Formulierungen von 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin nach intranasaler Gabe am Cynomolgus-Affen aus AUC Daten, normiert auf 1.0 mg/kg Körpergewicht.

**Tabelle 2:**

| **Formulierung** | **abs. Bioverfügbarkeit [%]** |
|---|---|
| wässrige Lösung, 1 Moläquivalent HCl | 4.8% |
| wässrige Lösung, 1.75 Moläquivalente HCl, mit Labrasol (1.5%) | 7.2% |
| wässrige Lösung, 1.75 Moläquivalent HCl, mit Labrasol (3%) | 8.2% |
| wässrige Lösung, 1.75 Moläquivalente HCl | 7.8% |
| Liposomen-Formulierung (5% Lipoid S100, 1 % Lysolecithin, 1.75 Moläquivalente HCl) . | 13.2% |

### (3) Beispiele

### Beispiel 1: wässrige Lösung; 10% Wirkstoff; 1.75 Moläquivalente HCl

| | |
|---|---|
| BIBN 4096 | 10 mg |
| 1N HCl | 20.45 mg |
| Mannitol | 5 mg |
| Wasser | ad 0.1 ml |

### Durchführung:

Die berechnete Menge Salzsäure wird in Wasser gegeben, der Wirkstoff unter Rühren und gegebenenfalls Erwärmen gelöst. Das Isotonans Mannitol wird zugegeben und die Lösung mit Wasser auf das Endvolumen aufgefüllt.

### Beispiel 2: wässrige Lösung; 25% Wirkstoff; 1.75 Moläquivalente HCl

| | |
|---|---|
| BIBN 4096 | 25 mg |
| 1N HCl | 51.12 mg |
| Mannitol | 5 mg |
| Wasser | ad 0.1 ml |

### Durchführung:

Die berechnete Menge Salzsäure wird in Wasser gegeben, der Wirkstoff unter Rühren und gegebenenfalls Erwärmen gelöst. Das Isotonans Mannitol wird zugegeben und die Lösung mit Wasser auf das Endvolumen aufgefüllt.

### Beispiel 3: wässrige Lösung; 20 % Wirkstoff, 1,5 % Labrasol; 1.75 Moläquivalente HCl

| | |
|---|---|
| BIBN 4096 | 20 mg |
| 1N HCl | 40.9 mg |
| Labrasol | 1.5 mg |
| Mannitol | 5 mg |
| Wasser | ad 0.1 ml |

### Durchführung:

Die berechnete Menge Salzsäure wird in Wasser gegeben, der Wirkstoff unter Rühren und gegebenenfalls Erwärmen gelöst. Das Isotonans Mannitol und das Labrasol werden zugegeben und die Lösung mit Wasser auf das Endvolumen aufgefüllt.

### Beispiel 4: wässrige Lösung; 10% Wirkstoff, 3 % Labrasol; 1.75 Moläquivalente HCl

| | |
|---|---|
| BIBN 4096 BS | 10 mg |
| 1N HCl | 20.45 mg |
| Labrasol | 3 mg |
| Mannitol | 5 mg |
| Wasser | ad 0.1 ml |

### Durchführung:

Die berechnete Menge Salzsäure wird in Wasser gegeben, der Wirkstoff unter Rühren und gegebenenfalls Erwärmen gelöst. Das Isotonans Mannitol und das Labrasol werden zugegeben und die Lösung mit Wasser mit Wasser auf das Endvolumen aufgefüllt.

### Beispiel 5: Liposomen; 5% Wirkstoff ; 1.75 Moläquivalente HCl

| | |
|---|---|
| BIBN 4096 | 5 mg |
| 1N HCl | 10.2 mg |
| Sojalecithin (Lipoid S 100) | 5 mg |
| Mannitol | 4.5 mg |
| Wasser | ad 0.1 ml |

### Durchführung:

Die berechnete Menge Salzsäure wird in Wasser gegeben, der Wirkstoff unter Rühren und gegebenenfalls Erwärmen gelöst. Das Isotonans Mannitol wird zugegeben, das Sojalecithin wird zugegeben. Nach Prädispergierung, z.B. mit einem Ultra-Turrax werden mittels Hochdruckhomogenisator mit bestimmter Zyklenzahl und bestimmtem Druck Liposomen erzeugt.

## Patentansprüche

1. Pharmazeutische Zusammensetzung für die nasale Applikation in Form einer wässrigen Lösung, umfassend
(a) 2% bis 25% g/v eines Wirkstoffs, ausgewählt aus der Gruppe (A) bis (CJ) bestehend aus
(A) 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,
(B) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,
(C) 1 -[N²-[4-Amino-3,5-dibrom-N-[[4-(3,4-dihydro-2(1 H)-oxochinazolin-3-yl)-1-piperidinyl]carbonyl]-D-phenylalanyl]-L-lysyl]-4-(4-pyridinyl)-piperazin,
(D) 1-[N²-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperidin,
(E) 1-[N²-[3,5-Dibrom-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazin,
(F) 1-[N2-[4-Amino-3,5-dibrom-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazin,
(G) 1-[3,5-Dibrom-N-[[4-(3,4-dihydro-2(1H)-oxothieno[3,4-d]pyrimidin-3-yl)-1-piperidinyl]carbonyl]-D-tyrosyl]-4-(1-piperidinyl)-piperidin,
(H) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidin,
(I) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,
(J) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,
(K) 1-[4-Amino-3,5-dibrom-N-[[4-(3,4-dihydro-2(1H)-oxothieno[3,2-d]pyrimidin-3-yl)-1-piperidinyl]carbonyl]-D-phenylalanyl]-4-(1-piperidinyl)-piperidin,
(L) 1-[4-Amino-3,5-dibrom-N-[[4-[1,3-dihydro-4-[3-(tri-fluoromethyl)phenyl]-2(2H)-oxoimidazol-1-yl]-1-piperidin-yl]carbonyl]-D-phenylalanyl]-4-(1-ethyl-4-piperidinyl)-piperidin,
(M) 1-[4-Amino-3,5-dibrom-N-[[4-[3,4-dihydro-2(1 H)-oxochinazolin-3-yl]-1-piperidinyl]carbonyl]-D-phenylalanyl]-4-(1-hexyl-4-piperidinyl)piperidin,
(N) 1-[4-Amino-3,5-dibrom-N-[[4-[3,4-dihydro-2(1 H)oxochinazolin-3-yl]-1-piperidinyl]carbonyl]-D-phenylalanyl]-4-(1-cyclopropylmethyl-4-piperidinyl)-piperidin,
(O) 1-[N-[[4-[3,4-Dihydro-2(1 H)-oxochinazolin-3-yl]-1-piperidinyl]carbonyl]-3-ethenyl-D,L-phenylalanyl]-4-(hexahydro-1 H-1-azepinyl)-piperidin,
(P) (R,S)-1-[4-[4-(3,4-Dihydro-2(1H)-oxochinazolin-3-yl)-1-piperidinyl]-2-[(4-hydroxy-3,5-dimethylphenyl)methyl]-1,4-dioxobutyl]-4-(1-piperidinyl)-piperidin,
(Q) 1-[4-Amino-3,5-dibrom-N-[[4-[N-(aminocarbonyl)-N-phenylamino]-1-piperidinyl]carbonyl]-D-phenylalanyl]-4-(1-piperidinyl)-piperidin,
(R) 1-[4-Amino-3,5-dibrom-N-[[4-(3,4-dihydro-2(1H)-oxochinazolin-3-yl)-1-piperidinyl]carbonyl]-D-phenylalanyl]-4-(5-methoxy-4-pyrimidinyl)-piperazin,
(S) 1-[4-Amino-3,5-dibrom-N-[[4-(1,1 -dioxido-3(4H)-oxo-1,2,4-benzothiadiazin-2-yl)-1-piperidinyl]carbonyl]-D-phenylalanyl]-4-(1-piperidinyl)-piperidin,
(T) 1-[4-Amino-3,5-dibrom-N-[[4-[2(1H)-oxochinolin-3-yl]-1-piperidinyl]-carbonyl]-D-phenylalanyl]-4-(1-piperidinyl)-piperidin,
(U) 1-[4-Amino-3,5-dibrom-N-[[4-[3,4-dihydro-2(1 H)-oxochinazolin-3-yl]-1-piperidinyl]carbonyl]-D-phenylalanyl]-4-[3-(dimethylamino)propyl]-piperazin,
(V) 1-[4-Amino-3,5-dibrom-N-[[4-[3,4-dihydro-2(1H)-oxochinazolin-3-yl]-1 -piperidinyl]carbonyl]-D-phenylalanyl]-4-(4-rnethyl-1-piperazinyl)-piperidin,
(W) 1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperidinyl)carbonyl]-piperazin,
(X) 1-[4-Amino-3,5-dibrom-N-[[4-[3,4-dihydro-2(1H)-oxochinazolin-3-yl]-1-piperidinyl]carbonyl]-P-phenylalanyl]-4-[(1-methyl-4-piperazinyl)-carbonyl]-piperazin,
(Y) 1-[4-Amino-3,5-dibrom-N-[[4-(3,4-dihydro-2(1H)-oxochinazolin-3-yl)-1-piperidinyl]carbonyl]-D-phenylalanyl]-4-[4-[4-(dimethylamino)butyl]-phenyl]-piperazin,
(Z) 1-[4-Amino-3,5-dibrom-N-[[4-(3,4-dihydro-2(1H)-oxochinazolin-3-yl)-1-piperidinyl]carbonyl]-D-phenylalanyl]-4-[4-(dimethylamino)-1-piperidinyl]-piperidin,
(AA) 1-[N²-[[4-(1,3-Dihydro-4-phenyl-2(2H)-oxoimidazol-1-yl)-1-piperidinyl]-carbonyl]-N'-methyl-D-tryptyl]-4-(4-methyl-1-piperazinyl)-piperidin,
(AB) 1-[N²-[[4-(1,3-Dihydro-4-phenyl-2(2H)-oxoimidazol-1-yl)-1-piperidinyl]-carbonyl]-N'-(1,1-dimethylethoxycarbonyl)-D-tryptyl]-4-(1-methyl-4-piperidinyl)-piperidin,
(AC) (R,S)-1-[4-[4-(3,4-Dihydro-2(1H)-oxochinazolin-3-yl)-1-piperidinyl]-2-[(3,5-dibro-4-methylphenyl)methyl]-1,4-dioxobutyl]-4-(4-methyl-1-piperazinyl)-piperidin,
(AD) (R,S)-1-[4-[4-(3,4-Dihydro-2(1H)-oxochinazolin-3-yl)-1-piperidinyl]-2-[(3,5-dibrom-4-methoxyphenyl)methyl]-1,4-dioxobutyl]-4-(1-methyl-4-piperidinyl)-piperidin,
(AE) (R,S)-1-[4-[4-(3,4-Dihydro-2(1H)-oxochinazolin-3-yl)-1-piperidinyl]-2-[(3,4-dibromphenyl)methyl]-1,4-dioxobutyl]-4-(4-methyl-1-piperazinyl)-piperidin.
(AF) 1-[N²-[N-[[4-(1,3-Dihydro-2(2H)-oxobenzimidazol-1-yl)-1-piperidinyl]-carbonyl]-3,5-dibrom-D-tyrosyl]-L-lysyl]-4-(4-pyridinyl)-piperazin,
(AG) 1-[4-Amino-3,5-dibrom-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidin,
(AH) 1-[N²-[4-Amino-3,5-dibrom-N-[[4-(1,3-dihydro-2(2H)-oxobenzimidazol-1-yl)-1-piperidinyl]carbonyl]D-phenylalanyl]-N⁶,N⁶-dimethyl-L-lysyl]-4-(4-pyridinyl)-piperazin,
(AI) 1-[N²-[4-Amino-3,5-dibrom-N-[[4-(3,4-dihydro-2(1H)-oxochinazolin-3-yl)-1-piperidinyl]carbonyl]-D-phenylalanyl]-N⁶,N⁶-dimethyl-L-lysyl]-4-(4-pyridinyl)-piperazin,
(AJ) (R,S)-1-[2-(4-Amino-3,5-dibrombenzoyl)-4-[4-(3,4-dihydro-2(1H)-oxo-chinazolin-3-yl)-1-piperidinyl]-4-oxobutyl]-4-(1-piperidinyl)-piperidin,
(AK) 1-[4-Amino-3,5-dibrom-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidin,
(AL) 1-[4-Amino-3,5-dibrom-N-[[4-[1,3-dihydro-2(2H)-oxoimidazo[4,5-c]chinolin-3-yl]-1-piperidinyl]carbonyl]-D-phenylalanyl]-4-(1-piperidinyl)carbonyl]-piperidin,
(AM) 1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,
(AN) 1-[N²-[3,5-Dibrom-N-[[4-(3,4-dihydro-2(1H)-oxochinazolin-3-yl)-1-piperidinyl]carbonyl]-D-tyrosyl]-N⁶,N⁶-dimethyl-L-lysyl]-4-(4-pyridinyl)-piperazin,
(AO) 1-[4-Amino-N-[[4-[4-(3-bromphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
(AP) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidin,
(AQ) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-piperidinyl)-piperidin,
(AR) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylatanyl]-4-(exo-8-methyl-8-azabicyclo[3,2,1]oct-3-yl)-piperazin,
(AS) 1-[3,5-Dibrom-N-[[4-(1,3-dihydro-4-phenyl-2(2H)-oxoimidazol-1-yl)-1-piperidinyl]carbonyl]-D-tyrosyl]-4-(1-piperidinyl)-piperidin,
(AT) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-ethyl-4-piperidinyl)-piperazin,
(AU) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(hexahydro-4-methyl-1H-1,4-diazepin-1-yl)piperidin,
(AV) 1-[3,5-Dibrom-N-[[4-(3,4-dihydro-2(1H)-oxochinazolin-3-yl)-1-piperidinyl]-carbonyl]-D-tyrosyl]-4-[1-(methylsulfonyl)-4-piperidinyl]-piperidin,
(AW) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidin,
(AX) 1-[3,5-Dibrom-N-[[4-[1,3-dihydro-4-[3-(trifluormethyl)phenyl]-2(2H)-oxo-imidazol-1-yl]-1-piperidinyl]carbonyl]-D-tyrosyl]-4-(hexahydro-1H-1-azepinyl)-piperidin,
(AY) 1-[3,5-Dibrom-N-[[4-(3,4-dihydro-2(1H)-oxochinazolin-3-yl)-1-piperidinyl]-carbonyl]-D-tyrosyl]-4-(1-methyl-4-piperidinyl)-piperidin,
(AZ) 1-[4-Amino-3,5-dibrom-N-[[4-(3,4-dihydro-2(1H)-oxochinazolin-3-yl)-1-piperidinyl]carbonyl]-D-phenylalanyl]-4-(exo-8-methyl-8-azabicyclo-[3,2,1]oct-3-yl)-piperazin,
(BA) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazin,
(BB) 1-[3,5-Dibrom-N-[[4-[1,3-dihydro-4-[3-(trifluormethyl)phenyl]-2(2H)-oxo-imidazol-1-yl]-1-piperidinyl]carbonyl]-D-tyrosyl]-4-(1-piperidinyl)piperidin,
(BC) 1-[N⁶-Acetyl-N²-[3,5-dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,
(BD) 1-[3,5-Dibrom-N-[[4-(1,3-dihydro-4-phenyl-2(2H)-oxoimidazol-1-yl)-1-piperidinyl]carbonyl]-D-tyrosyl]-4-(hexahydro-1H-1-azepinyl)-piperidin,
(BE) 1-[4-Amino-3,5-dibrom-N-[[4-(1,3-dihydro-4-(3-thienyl)-2(2H)-oxoimidazol-1-yl)-1-piperidinyl]carbonyl]-D-phenylatanyl]-4-(1-methyl-4-piperidinyl)-piperidin,
(BF) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperidin,
(BG) 1-[3,5-Dibrom-N-[[4-(3,4-dihydro-2(1H)-oxochinazolin-3-yl)-1-piperidinyl]-carbonyl]-D-tyrosyl]-4-[1-(hydroxycarbonylmethyl)-4-piperidinyl]-piperidin,
(BH) 1-[4-Amino-3,5-dibrom-N-[[4-[3,4-dihydro-2(1H)-oxochinazolin-3-yl]-1-piperidinyl]carbonyl]-D-phenylalanyl]-4-(1-methylsulfonyl-4-piperidinyl)-piperidin,
(BI) 1-[3,5-Dibrom-N-[[4-(3,4-dihydro-2(1H)-oxochinazolin-3-yl)-1-piperidinyl]-carbonyl]-D-tyrosyl]-4-(4-piperidinyl)-piperidin,
(BJ) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidin,
(BK) 1-[4-Amino-3,5-dibrom-N-[[4-(1,3-dihydro-4-(3-hydroxyphenyl)-2(2H)-oxo-imidazol-1-yl)-1-piperidinyl]carbonyl]-D-phenylalanyl]-4-(1-methyl-4-piperidinyl)-piperidin,
(BL) 1-[3,5-Dibrom-N-[[4-(3,4-dihydro-2(1H)-oxochinazolin-3-yl)-1-piperidinyl]-carbonyl]-D-tyrosyl]-4-(hexahydro-1 H-1-azepinyl)-piperidin,
(BM) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-piperidinyl)-piperidin,
(BN) 1-[4-Amino-3,5-dibrom-N-[[4-[4-(3-bromphenyl)-1,3-dihydro-2(2H)-oxo-imidazol-1-yl]-1-piperidinyl]carbonyl]-D-phenylalanyl]-4-(exo-8-methyl-8-azabicyclo[3,2,1]oct-3-yl)-piperazin,
(BO) 1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperidin,
(BP) 1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperazin,
(BQ) 1-[4-Amino-3,5-dibrom-N-[[4-[1,3-dihydro-4-(3-methoxyphenyl)-2(2H)-oxo-imidazol-1-yl]-1-piperidinyl]carbonyl]-D-phenylalanyl]-4-(exo-8-methyl-8-azabicyclo[3,2,1]oct-3-yl)-piperazin,
(BR) 1-[3,5-Dibrom-N-[[4-(3,4-dihydro-2(1H)-oxochinazolin-3-yl)-1-piperidinyl]-carbonyl]-D-tyrosyl]-4-[1-(cyclopropylmethyl)-4-piperidinyl]-piperidin,
(BS) 1-[4-Amino-3,5-Dibrom-N-[[4-(3,4-dihydro-2(1H)-oxochinazolin-3-yl)-1-piperidinyl]carbonyl]-D-phenylalanyl]-4-(hexahydro-1H-1-azepinyl)-piperidin,
(BT) 1-[4-Amino-3,5-dibrom-N-[[4-(3,4-dihydro-2(1 H)-oxochinazolin-3-yl)-1-piperidinyl]carbonyl]-D-phenylalanyl]-4-(4-piperidinyl)-piperidin,
(BU) 1-[3,5-Dibrom-N-[[4-(1,3-dihydro-4-phenyl-2(2H)-oxoimidazol-1-yl)-1 -piperidinyi]carbonyl]-D-tyrosyl]-4-(4-pyridinyl)-piperidin,
(BV) 1-[3,5-Dibrom-N-[[4-[1,3-dihydro-4-[3-(trifluormethyl)phenyl]-2(2H)-oxoimidazol-1-yl]-1-piperid inyl]carbonyl]-D-tyrosyl]-4-(1-methyl-4-piperid inyl)-piperazin,
(BW) 1-[N2-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-L-lysyl]-4-(4-pyridinyl)-piperazin,
(BX) 1-[3,5-Dibrom-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidin,
(BY) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,
(BZ) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(hexahydro-1H-1-azepinyl)-piperidin,
(CA) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperazin,
(CB) 1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(hexahydro-1 H-1-azepinyl)-piperidin,
(CC) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(4-pyridinyl)-piperazin,
(CD) 1-[3,5-Dibrom-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidin,
(CE) 1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazin,
(CF) 1-[3,5-Dibrom-N-[[4-[3,4-dihydro-2(1H)-oxochinazolin-3-yl]-1-piperidinyl]-carbonyl]-D-tyrosyl]-4-(1-methyl-4-piperidinyl)-piperazin ,
(CG) 1-[4-Amino-3,5-dibrom-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidin,
(CH) 1-[4-Amino-3,5-dibrom-N-[[4-[3,4-dihydro-2(1H)-oxochinazotin-3-yl]-1-piperidinyl]carbonyl]-D-phenylalanyl]-4-(1-pyrrolidinyl)-piperidin,
(CI) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(hexahydro-1 H-1-azepinyl)-piperidin und
(CJ) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazin, und
(b) 1.2 bis 2 Äquivalente einer physiologisch verträglichen, solubilisierenden Säure, wobei der Wirkstoff mittels der Säure in das entsprechende Salz übergeführt wird und gelöst vorliegt.

2. Pharmazeutische Zusammensetzung gemäß Anspruch 1, umfassend in wässriger Lösung 10 bis 20 Gew.% eines Wirkstoffs aus der Gruppe (A) bis (CJ).

3. Pharmazeutische Zusammensetzung gemäß Anspruch 1 oder 2, umfassend in wässriger Lösung 1.6 bis 1.9 Äquivalente einer physiologisch verträglichen, solubilisierenden Säure.

4. Pharmazeutische Zusammensetzung gemäß einem der Ansprüche 1 bis 3, **dadurch gekennzeichnet, dass** die verwendete physiologisch verträgliche, solubilisierende Säure ausgewählt ist aus der Gruppe bestehend aus Salzsäure, Phosphorsäure, Methansulfonsäure, Essigsäure, Ameisensäure und Bernsteinsäure.

5. Pharmazeutische Zusammensetzung gemäß einem der Ansprüche 1 bis 3, **dadurch gekennzeichnet, dass** die verwendete physiologisch verträgliche, solubilisierende Säure Salzsäure ist.

6. Pharmazeutische Zusammensetzung gemäß einem der Ansprüche 1 bis 5, zusätzlich enthaltend einen oder mehrere "absorption enhancer" in einer Konzentration von 0.1% bis 5% g/v.

7. Pharmazeutische Zusammensetzung gemäß einem der Ansprüche 1 bis 5, zusätzlich enthaltend ein gelbildendes Agens in einer Konzentration von 0.05% bis 1 % g/v zur Erhöhung der Viskosität.

8. Pharmazeutische Zusammensetzung gemäß einem der Ansprüche 1 bis 5, zusätzlich enthaltend ein liposomenbildendes Phospholipid in einer Konzentration von 2% bis 10% g/v und gegebenenfalls zusätzlich ein Hydrolyseprodukt eines liposomenbildenden Phospholipids in einer Konzentration von 0.5% bis 1 % g/v.

9. Pharmazeutische Zusammensetzung gemäß einem der vorangegangenen Ansprüche, abgefüllt unter Schutzgas als Einzeldosen in Primärpackmitteln, wobei die Primärpackmittel in eine Sekundärverpackung, ausgeführt als Beutel- oder Schlauchbeutelverpackung aus Aluminium, metallisierter Folie oder Transparent-Folie, unter Schutzgasatmosphäre, gegebenenfalls zusammen mit einem Sauerstoff-Fänger, eingebracht sind.

10. Pharmazeutische Zusammensetzung gemäß einem der vorangegangenen Ansprüche, **dadurch gekennzeichnet, dass** der Wirkstoff
1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin oder
1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin ist.

11. Verfahren zur Herstellung einer pharmazeutischen Zusammensetzung gemäß einem der vorangegangenen Ansprüche, umfassend die Schritte
(a) Auflösen von 2 bis 25% g/v, bezogen auf die pharmazeutische Zusammensetzung, eines Wirkstoffs ausgewählt aus der Gruppe (A) bis (CJ) gemäß Anspruch 1 in einer wässrigen Lösung, umfassend 1.2 bis 2 Äquivalente einer physiologisch verträglichen, solubilisierenden Säure oder
(b) Auflösen eines Salzes eines Wirkstoffs ausgewählt aus der Gruppe (A) bis (CJ) gemäß Anspruch 1, gebildet mit einer physiologisch verträglichen, solubilisierenden Säure, in Wasser in einer Menge, so dass der Wirkstoffgehalt der Zusammensetzung bezogen auf den Wirkstoff 2% bis 25% g/v beträgt, und
(c) Zusatz von 1.2 bis 2 Äquivalenten physiologisch verträglicher, solubilisierender Säure und
(d) optional Zusatz eines oder mehrerer Hilfsstoffe, ausgewählt aus absorption enhancern, gelbildenden Agentien und liposomenbildenden Phospholipiden sowie
(e) gegebenenfalls Verpackung der so erhaltenen Lösung unter Schutzgas als Einzeldosen in Primärpackmitteln, wobei die Primärpackmittel in eine Sekundärverpackung, ausgeführt als Beutel- oder Schlauchbeutelverpackung aus Aluminium, metallisierter Folie oder Transparent-Folie, unter Schutzgasatmosphäre, gegebenenfalls zusammen mit einem Sauerstoff-Fänger, eingebracht sind.

## Claims

1. Pharmaceutical composition for nasal application in the form of an aqueous solution, comprising
(a) 2% to 25 % w/v of an active substance, selected from the group (A) to (CJ) consisting of
(A) 1-[N²-[3,5-dibromo-N-[[4(3,4-dihydro-2(1H)-oxoquinazolin-3-yl)-1-piperidinyl]carbonyl]-D-tyrosyl]-L-lysyl]-4-(4-pyridinyl)-piperazine
(B) 1-[4-amino-3,5-dibromo-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidine
(C) 1-[N²-[4-amino-3,5-dibromo-N-[[4-(3,4-dihydro-2(1H)-oxoquinazolin-3-yl)-1-piperidinyl]carbonyl]-D-phenylalanyl]-L-lysyl]-4-(4-pyridinyl)-piperazine
(D) 1-[N²-[4-amino-3,5-dibromo-N-[[4-(3,4-dihydro-2(1H)-oxoquinazolin-3-yl)-1-piperidinyl]carbonyl]-D-phenylalanyl]-L-lysyl]-4-(4-pyridinyl)-piperidine,
(E) 1-[N²-[3,5-dibromo-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazine,
(F) 1-[N²-[4-amino-3,5-dibromo-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazine
(G) 1-[3,5-dibromo-N-[[4-(3,4-dihydro-2(1H)-oxothieno[3,4-d]pyrimidin-3-yl)-1-piperidinyl]carbonyl]-D-tyrosyl]-4-(1-piperidinyl)-piperidine
(H) 1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidine
(I) 1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidine
(J) 1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazine
(K) 1-[4-amino-3,5-dibromo-N-[[4-(3,4-dihydro-2(1 H)-oxothieno[3,2-d]pyrimidin-3-yl)-1-piperidinyl]carbonyl]-D-phenylalanyl]-4-(1-piperidinyl)-piperidine
(L) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-ethyl-4-piperidinyl)-piperidine
(M) 1-[4-amino-3,5-dibromo-N-[[4-[3,4-dihydro-2(1H)-oxoquinazolin-3-yl]-1-piperidinyl]carbonyl]-D-phenylalanyl]-4-(1-hexyl-4-piperidinyl)-piperidine
(N) 1-[4-amino-3,5-dibromo-N-[[4-[3,4-dihydro-2(1H)-oxoquinazolin-3-yl]-1-piperidinyl]carbonyl]-D-phenylalanyl]-4-(1-cyclopropylmethyl-4-piperidinyl)piperidine
(O) 1-[N-[[4-[3,4-dihydro-2(1H)-oxoquinazolin-3-yl]-1-piperidinyl]carbonyl]-3-ethenyl-D,L-phenylalanyl]-4-(hexahydro-1 H-1-azepinyl)-piperidine
(P) (R,S)-1-[4-[4-(3,4-dihydro-2(1 H)-oxoquinazolin-3-yl)-1-piperidinyl]-2-[(4-hydroxy-3,5-dimethylphenyl)methyl]-1,4-dioxobutyl]-4-(1-piperidinyl)-piperidine
(Q) 1-[4-amino-3,5-dibromo-N-[[4-[N-(aminocarbonyl)-N-phenylamino]-1-piperidinyl]carbonyl]-D-phenylalanyl]-4-(1-piperidinyl)-piperidine
(R) 1-[4-amino-3,5-dibromo-N-[[4-(3,4-dihydro-2(1H)-oxoquinazolin-3-yl)-1-piperidinyl]carbonyl]-D-phenylalanyl]-4-(5-methoxy-4-pyrimidinyl)-piperazine
(S) 1-[4-amino-3,5-dibromo-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidine
(T) 1-[4-amino-3,5-dibromo-N-[[4-[2(1H)-oxoquinolin-3-y1]-1-piperidinyl]carbonyl]-D-phenylalanyl]-4-(1-piperidinyl)-piperidine
(U) 1-[4-amino-3,5-dibromo-N-[[4-[3,4-dihydro-2(1H)-oxoquinazolin-3-yl]-1-piperidinyl]carbonyl]-D-phenylalanyl]-4-[3-(dimethylamino)propyl]-piperazine
(V) 1-[4-amino-3,5-dibromo-N-[[4-[3,4-dihydro-2(1H)-oxoquinazolin-3-yl]-1-piperidinyl]carbonyl]-D-phenylalanyl]-4-(4-methyl-1-piperazinyl)-piperidine
(W) 1-[4-amino-3,5-dibromo-N-[[4-[3,4-dihydro-2(1H)-oxoquinazolin-3-yl]-1-piperidinyl]carbonyl]-D-phenylalanyl]-4-[(1-methyl-4-piperidinyl)carbonyl]-piperazine
(X) 1-[4-amino-3,5-dibromo-N-[[4-[3,4-dihydro-2(1H)oxoquinazolin-3-yl]-1-piperidinyl]carbonyl]-D-phenylalanyl]-4-[(1-methyl-4-piperazinyl)carbonyl]-piperazine
(Y) 1-[4-amino-3,5-dibromo-N-[[4-(3,4-dihydro-2(1H)-oxoquinazolin-3-yl)-1-piperidinyl]carbonyl]-D-phenylalanyl]-4-[4-[4-(dimethylamino)butyl]phenyl]-piperazine
(Z) 1-[4-amino-3,5-dibromo-N-[[4-(3,4-dihydro-2(1H)-oxoquinazolin-3-yl)-1-piperidinyl]carbonyl]-D-phenylalanyl]-4-[4-(dimethylamino)-1-piperidinyl]-piperidine
(AA) 1-[N²-[[4-(1,3-dihydro-4-phenyl-2(2H)-oxoimidazol-1-y)-1-piperidinyl]carbonyl]-N'-methyl-D-tryptyl]-4-(4-methyl-1-piperazinyl)-piperidine
(AB) 1-[N²-[[4-(1,3-dihydro-4-phenyl-2(2H)-oxoimidazol-1-yl)-1-piperidinyl]camonyl]-N'-(1,1-dimethylethoxycarbonyl)-D-tryptyl]-4-(1-methyl-4-piperidinyl)-piperidine
(AC) (R,S)-1-[4-[4-(3,4-dihydro-2(1H)-oxoquinazolin-3-yl)-1-piperidinyl]-2-[(3,5-dibromo-4-methylphenyl)methyl]-1,4-dioxobutyl]-4-(4-methyl-1-piperazinyl)-piperidine
(AD) (R,S)-1-[4-[4-(3,4-dihydro-2(1H)-oxoquinazolin-3-yl)-1-piperidinyl]-2-[(3,5-dibromo-4-methoxyphenyl)methyl]-1,4-dioxobutyl]-4-(1-methyl-4-piperidinyl)-piperidine
(AE) (R,S)-1-[4-[4-(3,4-dihydro-2(1H)-oxoquinazolin-3-yl)-1-piperidinyl]-2-[(3,4-dibromophenyl)methyl]-1,4-dioxobutyl]-4-(4-methyl-1-piperazinyl)-piperidine
(AF) 1-[N²-[N-[[4-(1,3-dihydro-2(2H)-oxobenzimidazol-1-yl)-1-piperidinyl]carbonyl]-3,5-dibromo-D-tyrosyl]-L-lysyl]-4-(4-pyridinyl)-piperazine
(AG) 1-[4-amino-3,5-dibromo-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidine
(AH) 1-[N²-[4-amino-3,5-dibromo-N-[[4-(1,3-dihydro-2(2H)-oxobenzimidazol-1-yl)-1-piperidinyl]carbonyl]-D-phenylalanyl]-N⁶,N⁶-dimethyl-L-lysyl]-4-(4-pyridinyl)-piperazine
(AI) 1-[N²-[4-amino-3,5-dibromo-N-[[4-(3,4-dihydro-2(1H)-oxoquinazolin-3-yl)-1-piperidinyl]cartionyl]-D-phenylalanyl]-N⁶,N⁶-dimethyl-L-lysyl]-4-(4-pyridinyl)-piperazine
(AJ) (R,S)-1-[2-(4-amino-3,5-dibromobenzoyl)4-[4-(3,4-dihydro-2(1H)-oxoquinazolin-3-yl)-1-piperidinyl]-4-oxobutyl]-4-(1-piperidinyl)-piperidine
(AK) 1-[4-amino-3,5-dibromo-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidine
(AL) 1-[4-amino-3,5-dibromo-N-[[4-[1,3-dihydro-2(2H)-oxoimidazo[4,5-c]quinolin-3-yl]-1-piperidinyl]carbonyl]-D-phenylalanyl]-4-(1-piperidinyl)carbonyl]-piperidine
(AM) 1-[4-amino-3,5-dibromo-N-[[4-(3,4-dihydro-2(1H)-oxoquinazolin-3-yl)-1-piperidinyl]carbonyl]-D-phenylalanyl]-4-(1-pipendinyl)-piperidine
(AN) 1-[N²-[3,5-dibromo-N-[[4-(3,4-dihydro-2(1H)-oxoquinazolin-3-yl)-1-piperidinyl]carbonyl]-D-tyrosyl]-N⁶,N⁶-dimethyl-L-lysyl]-4-(4-pyridinyl)-piperazine
(AO) 1-[4-amino-N-[[4-[4-(3-bromophenyl)-1,3-dihydro-2(2H)-oxoimidazol-1-yl]-1-piperidinyl]carbonyl]-3,5-dibromo-D-phenylalanyl]-4-(1-methyl-4-piperidinyl)-piperidine
(AP) 1-[4-amino-3,5-dibromo-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidine
(AQ) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-piperidinyl)-piperidine
(AR) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(exo-8-methyl-8-azabicyclo[3,2,1]oct-3-yl)-piperazine
(AS) 1-[3,5-dibromo-N-[[4-(1,3-dihydro-4-phenyl-2(2H)-oxoimidazol-1-yl)-1-piperidinyl]carbonyl]-D-tyrosyl]-4-(1-piperidinyl)-piperidine
(AT) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-ethyl-4-piperidinyl)-piperazine
(AU) 1-[4-amino-3,5-dibromo-N-[[4-[1,3-dihydro-4-phenyl-2(2H)-oxoimidazol-1-yl]-1-piperidinyl]carbonyl]-D-phenylalanyl]-4-(hexahydro-4-methyl-1H-1,4-diazepine-1-yl)piperidine
(AV) 1-[3,5-dibromo-N-[[4-(3,4-dihydro-2(1H)-oxoquinazolin-3-yl)-1-piperidinyl]carbonyl]-D-tyrosyl]-4-[1-(methylsulphonyl)-4-piperidinyl]-piperidine
(AW) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidine
(AX) 1-[3,5-dibromo-N-[[4-[1,3-dihydro-4-[3-(trifluoromethyl)phenyl]-2(2H)-oxoimidazol-1-yl]-1-piperidinyl]carbonyl]-D-tyrosyl]-4-(hexahydro-1 H-1-azepinyl)-piperidine
(AY) 1-[3,5-dibromo-N-[[4-(3,4-dihydro-2(1H)-oxoquinazolin-3-yl)-1-piperidinyl]carbonyl]-D-tyrosyl]-4-(1-methyl-4-piperidinyl)-piperidine
(AZ) 1-[4-amino-3,5-dibromo-N-[[4-(3,4-dihydro-2(1 H)-oxoquinazolin-3-yl)-1-piperidinyl]carbonyl]-D-phenylalanyl]-4-(exo-8-methyl-8-azabicyclo[3,2,1]oct-3-yl)-piperazine
(BA) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazine
(BB) 1-[3,5-dibromo-N-[[4-[1,3-dihydro-4-[3-(trifluoromethyl)phenyl]-2(2H)oxoimidazol-1-yl]-1-piperidinyl]carbonyl]-D-tyrosyl]-4-(1-piperidinyl)piperidine
(BC) 1-[N⁶-acetyl-N²-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine
(BD) 1-[3,5-dibromo-N-[[4-(1,3-dihydro-4-phenyl-2(2H)-oxoimidazol-1-yl)-1-piperidinyl]carbonyl]-D-tyrosyl]-4-(hexahydro-1H-1-azepinyl)-piperidine
(BE) 1-[4-amino-3,5-dibromo-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidine
(BF) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-methyl-4-piperidinyl)-piperidine
(BG) 1-[3,5-dibromo-N-[[4-(3,4-dihydro-2(1H)-oxoquinazolin-3-yl)-1-piperidinyl]carbonyl]-D-tyrosyl]-4-[1-(hydroxycarbonylmethyl)-4-piperidinyl]-piperidine
(BH) 1-[4-amino-3,5-dibromo-N-[[4-[3,4-dihydro-2(1H)-oxoquinazolin-3-yl]-1-piperidinyl]carbonyl]-D-phenylalanyl]-4-(1-methylsulphonyl-4-piperidinyl)-piperidine
(BI) 1-[3,5-dibromo-N-[[4-(3,4-dihydro-2(1H)-oxoquinazolin-3-yl)-1-piperidinyl]carbonyl]-D-tyrosyl]-4-(4-piperidinyl)-piperidine
(BJ) 1-[4-amino-3,5-dibromo-N-[[4-(1,3-dihydro-4-phenyl-2(2H)-oxoimidazol-1-yl)-1-piperidiny]carbonyl]-D-phenylalanyl]-4-(1-ethyl-4-piperidinyl)-piperidine
(BK) 1-[4-amino-3,5-dibromo-N-[[4-(1,3-dihydro-4-(3-hydroxyphenyl)-2(2H)-oxoimidazol-1-yl)-1-piperidinyl]carbonyl]-D-phenylalanyl]-4-(1-methyl-4-piperidinyl)-piperidine
(BL) 1-[3,5-dibromo-N-[[4-(3,4-dihydro-2(1H)-oxoquinazolin-3-yl)-1-piperidinyl]carbonyl]-D-tyrosyl]-4-(hexahydro-1H-1-azepinyl)-piperidine
(BM) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-piperidinyl)-piperidine
(BN) 1-[4-amino-3,6-dibromo-N-[[4-[4-(3-bromophenyl)-1,3-dihydro-2(2H)-oxoimidazol-1-yl]-1-piperidinyl]carbonyl]-D-phenylalanyl]-4-(exo-8-methyl-8-azabicyclo[3,2,1]oct-3-yl)-piperazine
(BO) 1-[4-amino-3,5-dibromo-N-[[4-(3,4-dihydro-2(1H)-oxoquinazolin-3-yl)-1-piperidinyl]carbonyl]-D-phenylalanyl]-4-(1-ethyl-4-piperidinyl)-piperidine
(BP) 1-[4-amino-3,5-dibromo-N-[[4-(3,4-dihydro-2(1H)-oxoquinazolin-3-yl)-1-piperidinyl]carbonyl]-D-phenylalanyl]-4-(1-ethyl-4-piperidinyl)-piperazine
(BQ) 1-[4-amino-3,5-dibromo-N-[[4-[1,3-dihydro-4-(3-methoxyphenyl)-2(2H)-oxoimidazol-1-yl]-1-piperidinyl]carbonyl]-D-phenylalanyl]-4-(exo-8-methyl-8-azabicyclo[3,2,1]oct-3-yl)-piperazine
(BR) 1-[3,5-dibromo-N-[[4-(3,4-dihydro-2(1H)-oxoquinazolin-3-yl)-1-piperidinyl]carbonyl]-D-tyrosyl]-4-[1-(cyclopropylmethyl)-4-piperidinyl]-piperidine
(BS) 1-[4-amino-3,5-dibromo-N-[[4-(3,4-dihydro-2(1H)-oxoquinazolin-3-yl)-1-pipendinyl]carbonyl]-D-phenylalanyl]-4-(hexahydro-1H-1-azepinyl)-piperidine
(BT) 1-[4-amino-3,5-dibromo-N-[[4-(3,4-dihydro-2(1H)-oxoquinazolin-3-yl)-1-piperidinyl]carbonyl]-D-phenylalanyl]-4-(4-piperídinyl)-piperidine
(BU) 1-[3,5-dibromo-N-[[4-(1,3-dihydro-4-phenyl-2(2H)-oxoimidazol-1-yl)-1-piperidinyl]carbonyl]-D-tyrosyl]-4-(4-pyridinyl)-piperidine
(BV) 1-[3,5-dibromo-N-[[4-[1,3-dihydro-4-[3-(trifluoromethyl)phenyl]-2(2H)-oxoimidazol-1-yl]-1-piperidinyl]carbonyl]-D-tyrosyl]-4-(1-methyl-4-piperidinyl)-piperazine
(BW) 1-[N2-[3,5-dibromo-N-[[4-[1,3-dihydro-4-[3-(trifluoromethyl)phenyl]-2(2H)-oxoimidazol-1-yl]1-piperidinyl]carbonyl]-D-tyrosyl]-L-lysyl]-4-(4-pyridinyl)-piperazine
(BX) 1-[3,5-dibromo-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidine
(BY) 1-[4-amino-N-[[4-[4-(3-chlorophenyl)-1,3-dihydro-2(2H)-oxoimidazol-1-yl]-1-piperidinyl]carbonyl]-3,5-dibromo-D-phenylalanyl]-4-(1-methyl-4-piperdinyl)-piperidine
(BZ) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(hexahydro-1H-1-azepinyl)-piperidine
(CA) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-methyl-4-piperidinyl)-piperazine
(CB) 1-[4-amino-N-[[4-[4-(3-chlorophenyl)-1,3-dihydro-2(2H)-oxoimidazol-1-yl]-1-piperidinyl]carbonyl]-3,5-dibromo-D-phenylalanyl]-4-(hexahydro-1H-1-azepinyl)-piperidine
(CC) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(4-pyridinyl)-piperazine
(CD) 1-[3,5-dibromo-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidine
(CE) 1-[4-amino-3,5-dibmmo-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazine
(CF) 1-[3,5-dibromo-N-[[4-[3,4-dihydro-2(1H)-oxoquinazolin-3-yl]-1-piperidinyl]carbonyl]-D-tyrosyl]-4-(1-methyl-4-piperidinyl)-piperazine
(CG) 1-[4-amino-3,5-dibromo-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidine
(CH) 1-[4-amino-3,5-dibromo-N-[[4-[3,4-dihydro-2(1H)-oxoquinazolin-3-yl]-1-piperidinyl]carbonyl]-D-phenylalanyl]-4-(1-pyrrolidinyl)-piperidine
(CI) 1-[4-amino-3,5-dibromo-N-[[4-(1,3-dihydro-4-phenyl-2(2H)-oxoimidazoi-1-yl)-1-piperidinyl]carbonyl]-D-phenylalanyl]-4-(hexahydro-1 H-1-azepinyl)-piperidine and
(CJ) 1-[4-amino-3,5-dibromo-N-[[4-(1,3-dihydro-4-(3-thienyl)-2(2H)-oxoimidazol-1-yl)-1-piperidinyl]carbonyl]-D-phenylalanyl]-4-(1-methyl-4-piperdinyl)-piperazine, and
(b) 1.2 to 2 equivalents of a physiologically acceptable solubilising acid, the active substance being, converted by means of the acid into the corresponding salt and being dissolved.

2. Pharmaceutical composition according to claim 1, comprising in aqueous solution 10 to 20 wt.% of an active substance from the group (A) to (CJ).

3. Pharmaceutical composition according to claim 1 or 2, comprising in aqueous solution 1.6 to 1.9 equivalents of a physiologically acceptable solubilising acid.

4. Pharmaceutical composition according to one of claims 1 to 3, **characterised in that** the physiologically acceptable solubilising acid used is selected from the group consisting of hydrochloric acid, phosphoric acid, methanesulphonic acid, acetic acid, formic acid and succinic acid.

5. Pharmaceutical composition according to one of claims 1 to 3, **characterised in that** the physiologically acceptable solubilising acid used is hydrochloric acid.

6. Pharmaceutical composition according to one of claims 1 to 5, additionally containing one or more absorption enhancer(s) in a concentration of 0.1 % to 5% w/v.

7. Pharmaceutical composition according to one of claims 1 to 5, additionally containing a gel-forming agent in a concentration of 0.05% to 1% w/v to increase the viscosity.

8. Pharmaceutical composition according to one of claims 1 to 5, additionally containing a liposome-forming phospholipid in a concentration of 2% to 10% w/v and optionally additionally a hydrolysis product of a liposome-forming phospholipid in a concentration of 0.5% to 1% w/v.

9. Pharmaceutical composition according to one of the preceding claims, transferred under protective gas as individual doses into primary packaging means, the primary packaging means being placed in a secondary package in the form of a bag or tubular bag package made of aluminium, metallised film or transparent film, under a protective gas atmosphere, optionally together with an oxygen absorber.

10. Pharmaceutical composition according to one of the preceding claims, **characterised in that** the active substance is
1-[N²-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine or
1-[4-amino-3,5-dibromo-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidine.

11. Process for preparing a pharmaceutical composition according to one of the preceding claims, comprising the steps of
(a) dissolving 2 to 25% w/v, based on the pharmaceutical composition, of an active substance selected from the group (A) to (CJ) according to claim 1 in an aqueous solution, comprising 1.2 to 2 equivalents of a physiologically acceptable solubilising acid or
(b) dissolving a salt of an active substance selected from the group (A) to (CJ) according to claim 1, formed with a physiologically acceptable solubilising acid, in water in an amount such that the active substance content of the composition based on the active substance is 2% to 25% w/v, and
(c) adding 1.2 to 2 equivalents of physiologically acceptable solubilising acid and
(d) optionally adding one or more excipients, selected from absorption enhancers, gel-forming agents and liposome-forming phospholipids as well as
(e) optionally packaging the resulting solution under protective gas as single doses in primary packaging means, the primary packaging means being placed in a secondary package in the form of a bag or tubular bag packaging made of aluminium, metallised film or transparent film, under a protective gas atmosphere, optionally together with an oxygen absorber.

## Revendications

1. Composition pharmaceutique pour l'administration intranasale sous forme d'une solution aqueuse, comprenant:
(a) 2 % à 25 % en masse/volume (m/v) d'une substance active choisie dans le groupe de (A) à (CJ) constitué de
(A) la 1-[N²-[3,5-dibromo-N-[[4-(3,4-dihydro-2(1H)-oxoquinazolin-3-yl)-1-pipéridinyl] carbonyl]-D-tyrosyl]-L-lysyl]-4-(4-pyridinyl)pipérazine,
(B) la 1-[4-amino-3,5-dibromo-N-[[4-(2,3,4,5-tétrahydro-2(1H)-oxo-1,3-benzodiazépin-3-yl)-1-pipéridinyl]carbonyl]-D-phénylalanyl]-4-(1-pipéridinyl)pipéridine,
(C) la 1-[N²-[4-amino-3,5-dibromo-N-[[4-(3,4-dihydro-2(1H)-oxoquinazolin-3-yl)-1-pipéridinyl]carbonyl]-D-phénylalanyl]-L-lysyl]-4-(4-pyridinyl)-pipérazine,
(D) la 1-[N²-[4-amino-3,5-dibromo-N-[[4-(3,4-dihydro-2(1H)-oxoquinazolin-3-yl)-1-pipéridinyl]carbonyl]-D-phénylalanyl]-L-lysyl]-4-(4-pyridinyl)-pipéridine,
(E) la 1-[N²-[3,5-dibromo-N-[[4-(1,3-dihydro-4-phényl-2(2H)-oxoimidazol-1-yl)-1-pipéridinyl]carbonyl]-D-tyrosyl]-L-lysyl]-4-(4-pyridinyl)-pipérazine,
(F) la 1-[N²-[4-amino-3,5-dibromo-N-[[4-(1,3-dihydro-4-phényl-2(2H)-oxoimidazol-1-yl)-1-pipéridinyl]carbonyl]-D-phénylalanyl]-L-lysyl]-4-(4-pyridinyl)pipérazine,
(G) la 1-[3,5-dibromo-N-[[4-(3,4-dihydro-2(1H)-oxothiéno[3,4-d]pyrimidin-3-yl)-1-pipéridinyl]carbonyl]-D-tyrosyl]-4-(1-pipéridinyl)pipéridine,
(H) la 1-[4-amino-3,5-dibromo-N-[[4-(2,4-dihydro-5-phényl-3(3H)-oxo-1,2,4-triazol-2-yl)-1-pipéridinyl] carbonyl]-D-phénylalanyl]-4-(1-méthyl-4-pipéridinyl)pipéridine,
(I) la 1-[4-amino-3,5-dibromo-N-[[4-(2,4-dihydro-S-phényl-3(3H)-oxo-1,2,4-triazol-2-yl)-1-pipéridinyl]carbonyl]-D-phénylalanyl]-4-(1-pipéridinyl)pipéridine,
(J) la 1-[4-amino-3,5-dibromo-N-[[4-(2,4-dihydro-5-phényl-3(3H)-oxo-1,2,4-triazol-2-yl)-1-pipéridinyl] carbonyl]-D-phénylalanyl]-4-(1-méthyl-4-pipéridinyl)pipérazine,
(K) la 1-[4-amino-3,5-dibromo-N-[[4-(3,4-dihydro-2(1H)-oxothiéno[3,2-d]pyrimidin-3-yl)-1-pipéridinyl]carbonyl]-D-phénylalanyl]-4-(1-pipéridinyl)pipéridine,
(L) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(1-éthyl-4-pipéridinyl)pipéridine,
(M) la 1-[4-amino-3,5-dibromo-N-[[4-[3,4-dihydro-2(1H)-oxoquinazolin-3-yl]-1-pipéridinyl]carbonyl]-D-phénylalanyl]-4-(1-hexyl-4-pipéridinyl)-pipéridine,
(N) la 1-[4-amino-3,5-dibromo-N-[[4-[3,4-dihydro-2(1H)-oxoquinazolin-3-yl]-1-pipéridinyl]carbonyl]-D-phénylalanyl]-4-(1-cyclopropylméthyl-4-pipéridinyl)pipéridine,
(O) la 1-[N-[[4-[3,4-dihydro-2(1H)-oxoquinazolin-3-yl]-1-pipéridinyl]-carbonyl]-3-éthényl-D,L-phénylalanyl]-4-(hexahydro-1H-1-azépinyl)-pipéridine,
(P) la (R,S)-1-[4-[4-(3,4-dihydro-2(1H)-oxoquinazolin-3-yl)-1-pipéridinyl]-2-[(4-hydroxy-3,5-diméthylphényl)méthyl]-1,4-dioxobutyl]-4-(1-pipéridinyl)pipéridine,
(Q) la 1-[4-amino-3,5-dibromo-N-[[4-[N-(aminocarbonyl)-N-phénylamino]-1-pipéridinyl]carbonyl]-D-phénylalanyl]-4-(1-pipéridinyl)pipéridine,
(R) la 1-[4-amino-3,5-dibromo-N-[[4-(3,4-dihydro-2(1H)-oxoquinazolin-3-yl)-1-pipéridinyl]carbonyl]-D-phénylalanyl]-4-(5-méthoxy-4-pyrimidinyl)pipérazine,
(S) la 1-[4-amino-3,5-dibromo-N-[[4-(1,1-dioxydo-3(4H)-oxo-1,2,4-benzothiadiazin-2-yl)-1-pipéridinyl]carbonyl]-D-phénylalanyl]-4-(1-pipéridinyl)pipéridine,
(T) la 1-[4-amino-3,5-dibromo-N-[[4-[2(1H)-oxoquinoléin-3-yl]-1-pipéridinyl]carbonyl]-D-phénylalanyl]-4-(1-pipéridinyl)pipéridine,
(U) la 1-[4-amino-3,5-dibromo-N-[[4-[3,4-dihydro-2(1H)-oxoquinazolin-3-yl]-1-pipéridinyl]carbonyl]-D-phénylalanyl]-4-[3-(diméthylamino)-propyl]pipérazine,
(V) la 1-[4-amino-3,5-dibromo-N-[[4-[3,4-dihydro-2(1H)-oxoquinazolin-3-yl]-1-pipéridinyl]carbonyl]-D-phénylalanyl]-4-(4-méthyl-1-pipérazinyl)-pipéridine,
(W) la 1-[4-amino-3,5-dibromo-N-[[4-[3,4-dihydro-2(1H)-oxoquinazolin-3-yl]-1-pipéridinyl]carbonyl]-D-phénylalanyl]-4-[(1-méthyl-4-pipéridinyl)-carbonyl]pipérazine,
(X) la 1-[4-amino-3,5-dibromo-N-[[4-[3,4-dihydro-2(1H)-oxoquinazolin-3-yl]-1-pipéridinyl]carbonyl]-D-phénylalanyl]-4-[(1-méthyl-4-pipérazinyl)-carbonyl]pipérazine,
(Y) la 1-[4-amino-3,5-dibromo-N-[[4-(3,4-dihydro-2(1H)-oxoquinazolin-3-yl)-1-pipéridinyl]carbonyl]-D-phénylalanyl]-4-[4-[4-(diméthylamino)butyl]phényl]pipérazine,
(Z) la 1-[4-amino-3,5-dibromo-N-[[4-(3,4-dihydro-2(1H)-oxoquinazolin-3-yl)-1-pip éridinyl] carbonyl]-D-phénylalanyl]-4-[4-(diméthylamino)-1-pipéridinyl]pipéridine,
(AA) la 1-[N²-[[4-(1,3-dihydro-4-phényl-2(2H)-oxoimidazol-1-yl)-1-pipéridinyl] carbonyl]-N'-méthyl-D-tryptyl]-4-(4-méthyl-1-pipérazinyl)-pipéridine,
(AB) la 1-[N²-[[4-(1,3-dihydro-4-phényl-2(2H)-oxoimidazol-1-yl)-1-pipéridinyl]carbonyl]-N'-(1,1-diméthyléthoxycarbonyl)-D-tryptyl]-4-(1-méthyl-4-pipéridinyl)pipéridine,
(AC) la (R,S)-1-[4-[4-(3,4-dihydro-2(1H)-oxoquinazolin-3-yl)-1-pipéridinyl]-2-[(3,5-dibromo-4-méthylphényl)méthyl]-1,4-dioxobutyl]-4-(4-méthyl-1-pipérazinyl)pipéridine,
(AD) la (R,S)-1-[4-[4-(3,4-dihydro-2(1H)-oxoquinazolin-3-yl)-1-pipéridinyl]-2-[(3,5-dibromo-4-méthoxyphényl)méthyl]-1,4-dioxobutyl]-4-(1-méthyl-4-pipéridinyl)pipéridine,
(AE) la (R,S)-1-[4-[4-(3,4-dihydro-2(1H)-oxoquinazolin-3-yl)-1-pipéridinyl]-2-[(3,4-dibromophényl)méthyl]-1,4-dioxobutyl]-4-(4-méthyl-1-pipérazinyl)pipéridine,
(AF) la 1-[NZ-[N-[[4-(1,3-dihydro-2(2H)-oxobenzimidazol-1-yl)-1-pipéridinyl]carbonyl]-3,5-dibromo-D-tyrosyl]-L-lysyl]-4-(4-pyridinyl)pipérazine,
(AG) la 1-[4-amino-3,5-dibromo-N-[[4-(1,3-dihydro-6-hydroxy-2(2H)-oxobenzimidazol-1-yl)-1-pipéridinyl]carbonyl]-D-phénylalanyl]-4-(1-pipéridinyl)pipéridine,
(AH) la 1-[N²-[4-amino-3,5-dibromo-N-[[4-(1,3-dihydro-2(2H)-oxobenzimidazol-1-yl)-1-pipéridinyl]carbonyl]-D-phénylalanyl]-N⁶,N⁶-diméthyl-L-lysyl]-4-(4-pyridinyl)pipérazine,
(AI) la 1-[N²-[4-amino-3,5-dibromo-N-[[4-(3,4-dihydro-2(1H)-oxoquinazolin-3-yl)-1-pipéridinyl]carbonyl]-D-phénylalanyl]-N⁶,N⁶-diméthyl-L-lysyl]-4-(4-pyridinyl)pipérazine,
(AJ) la (R,S)-1-[2-(4-amino-3,5-dibromobenzoyl)-4-[4-(3,4-dihydro-2(1H)-oxoquinazolin-3-yl)-1-pipéridinyl]-4-oxobutyl]-4-(1-pipéridinyl)-pipéridine,
(AK) la 1-[4-amino-3,5-dibromo-N-[[4-(3,4-dihydro-2,2-dioxydo-2,1,3-benzothiadiazin-3-yl)-1-pipéridinyl]carbonyl]-D-phénylalanyl]-4-(1-pipéridinyl)pipéridine,
(AL) la 1-[4-amino-3,5-dibromo-N-[[4-[1,3-dihydro-2(2H)-oxoimidazo[4,5-c]quinoléin-3-yl]-1-pipéridinyl]carbonyl]-D-phénylalanyl]-4-(1-pipéridinyl)carbonyl]pipéridine,
(AM) la 1-[4-amino-3,5-dibromo-N-[[4-(3,4-dihydro-2(1H)-oxoquinazolin-3-yl)-1-pipéridinyl]carbonyl]-D-phénylalanyl]-4-(1-pipéridinyl)pipéridine,
(AN) la 1-[N²-[3,5-dibromo-N-[[4-(3,4-dihydro-2(1H)-oxoquinazolin-3-yl)-1-pipéridinyl]carbonyl]-D-tyrosyl]-N⁶,N⁶-diméthyl-L-lysyl]-4-(4-pyridinyl)pipérazine,
(AO) la 1-[4-amino-N-[[4-[4-(3-bromophényl)-1,3-dihydro-2(2H)-oxoimidazol-1-yl]-1-pipéridinyl]carbonyl]-3,5-dibromo-D-phénylalanyl]-4-(1-méthyl-4-pipéridinyl)pipéridine,
(AP) la 1-[4-amino-3,5-dibromo-N-[[4-[1,3-dihydro-4-phényl-2(2H)-oxoimidazol-1-yl]-1-pipéridinyl]carbonyl]-D-phénylalanyl]-4-(4-méthyl-1-pipérazinyl)pipéridine,
(AQ) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(1-pipéridinyl)pipéridine,
(AR) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(exo-8-méthyl-8-azabicyclo[3,2,1]oct-3-yl)pipérazine,
(AS) la 1-[3,5-dibromo-N-[[4-(1,3-dihydro-4-phényl-2(2H)-oxoimidazol-1-yl)-1-pipéridinyl]carbonyl]-D-tyrosyl]-4-(1-pipéridinyl)pipéridine,
(AT) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(1-éthyl-4-pipéridinyl)pipérazine,
(AU) la 1-[4-amino-3,5-dibromo-N-[[4-[1,3-dihydro-4-phényl-2(2H)-oxoimidazol-1-yl]-1-pipéridinyl]carbonyl]-D-phénylalanyl]-4-(hexahydro-4-méthyl-1H-1,4-diazépin-1-yl)pipéridine,
(AV) la 1-[3,5-dibromo-N-[[4-(3,4-dihydro-2(1H)-oxoquinazolin-3-yl)-1-pipéridinyl]carbonyl]-D-tyrosyl]-4-[1-(méthylsulfonyl)-4-pipéridinyl]-pipéridine,
(AW) la 1-[4-amino-3,5-dibromo-N-[[4-(1,3-dihydro-4-phényl-2(2H)-oxoimidazol-1-yl)-1-pipéridinyl] carbonyl]-D-phénylalanyl]-4-(1-méthyl-4-pipéridinyl)pipéridine,
(AX) la 1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(hexahydro-1H-1-azépinyl)pipéridine,
(AY) la 1-[3,5-dibromo-N-[[4-(3,4-dihydro-2(1H)-oxoquinazolin-3-yl)-1-pipéridinyl]carbonyl]-D-tyrosyl]-4-(1-méthyl-4-pipéridinyl)pipéridine,
(AZ) la 1-[4-amino-3,5-dibromo-N-[[4-(3,4-dihydro-2(1H)-oxoquinazolin-3-yl)-1-pipéridinyl]carbonyl]-D-phénylalanyl]-4-(exo-8-méthyl-8-azabicyclo-[3,2,1]oct-3-yl)pipérazine,
(BA) la 1-[4-amino-3,5-dibromo-N-[[4-(1,3-dihydro-4-phényl-2(2H)-oxoimidazol-1-yl)-1-pipéridinyl]carbonyl]-D-phénylalanyl]-4-(1-méthyl-4-pipéridinyl)pipérazine,
(BB) la 1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(1-pipéridinyl)pipéridine,
(BC) la 1-[N⁶-acétyl-N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)pipérazine,
(BD) la 1-[3,5-dibromo-N-[[4-(1,3-dihydro-4-phényl-2(2H)-oxoimidazol-1-yl)-1-pipéridinyl]carbonyl]-D-tyrosyl]-4-(hexahydro-1H-1-azépinyl)-pipéridine,
(BE) la 1-[4-amino-3,5-dibromo-N-[[4-(1,3-dihydro-4-(3-thiényl)-2(2H)-oxoimidazol-1-yl)-1-pipéridinyl]carbonyl]-D-phénylalanyl]-4-(1-méthyl-4-pipéridinyl)pipéridine,
(BF) la 1-[4-amino-3,5-dibromo-N-[[4-[1,3-dihydro-4-[3-(trifluorométhyl)-phényl] -2(2H)-oxoimidazol-1-yl]-1-pipéridinyl] carbonyl] -D-phénylalanyl]-4-(1-méthyl-4-pipéridinyl)pipéridine,
(BG) la 1-[3,5-dibromo-N-[[4-(3,4-dihydro-2(1H)-oxoquinazolin-3-yl)-1-pipéridinyl]carbonyl]-D-tyrosyl]-4-[1-(hydroxycarbonylméthyl)-4-pipéridinyl]pipéridine,
(BH) la 1-[4-amino-3,5-dibromo-N-[[4-[3,4-dihydro-2(1H)-oxoquinazolin-3-yl]-1-pipéridinyl]carbonyl]-D-phénylalanyl]-4-(1-méthylsulfonyl-4-pipéridinyl)pipéridine,
(BI) la 1-[3,5-dibromo-N-[[4-(3,4-dihydro-2(1H)-oxoquinazolin-3-yl)-1-pipéridinyl]carbonyl]-D-tyrosyl]-4-(4-pipéridinyl)pipéridine,
(BJ) la 1-[4-amino-3,5-dibromo-N-[[4-(1,3-dihydro-4-phényl-2(2H)-oxoimidazol-1-yl)-1-pipéridinyl] carbonyl]-D-phénylalanyl]-4-(1-éthyl-4-pipéridinyl)pipéridine,
(BK) la 1-[4-amino-3,5-dibromo-N-[[4-(1,3-dihydro-4-(3-hydroxyphényl)-2(2H)-oxoimidazol-1-yl)-1-pipéridinyl]carbonyl]-D-phénylalanyl]-4-(1-méthyl-4-pipéridinyl)pipéridine,
(BL) la 1-[3,5-dibromo-N-[[4-(3,4-dihydro-2(1H)-oxoquinazolin-3-yl)-1-pipéridinyl]carbonyl]-D-tyrosyl]-4-(hexahydro-1H-1-azépinyl)pipéridine,
(BM) la 1-[4-amino-3,5-dibromo-N-[[4-(1,3-dihydro-4-phényl-2(2H)-oxoimidazol-1-yl)-1-pipéridinyl]carbonyl]-D-phénylalanyl]-4-(1-pipéridinyl)pipéridine,
(BN) la 1-[4-amino-3,5-dibromo-N-[[4-[4-(3-bromophényl)-1,3-dihydro-2(2H)-oxoimidazol-1-yl]-1-pipéridinyl]carbonyl]-D-phénylalanyl]-4-(exo-8-méthyl-8-azabicyclo[3,2,1]oct-3-yl)pipérazine,
(BO) la 1-[4-amino-3,5-dibromo-N-[[4-(3,4-dihydro-2(1H)-oxoquinazolin-3-yl)-1-pipéridinyl]carbonyl]-D-phénylalanyl]-4-(1-éthyl-4-pipéridinyl)pipéridine,
(BP) la 1-[4-amino-3,5-dibromo-N-[[4-(3,4-dihydro-2(1H)-oxoquinazolin-3-yl)-1-pipéridinyl]carbonyl]-D-phénylalanyl]-4-(1-éthyl-4-pipéridinyl)-pipérazine,
(BQ) la 1-[4-amino-3,5-dibromo-N-[[4-[1,3-dihydro-4-(3-méthoxyphényl)-2(2H)-oxoimidazol-1-yl]-1-pipéridinyl]carbonyl]-D-phénylalanyl]-4-(exo-8-méthyl-8-azabicyclo[3,2,1]oct-3-yl)pipérazine,
(BR) la 1-[3,5-dibromo-N-[[4-(3,4-dihydro-2(1H)-oxoquinazolin-3-yl)-1-pipéridinyl]carbonyl]-D-tyrosyl]-4-[1-(cyclopropylméthyl)-4-pipéridinyl]pipéridine,
(BS) la 1-[4-amino-3,5-dibromo-N-[[4-(3,4-dihydro-2(1H)-oxoquinazolin-3-yl)-1-pipéridinyl]carbonyl]-D-phénylalanyl]-4-(hexahydro-1H-1-azépinyl)pipéridine,
(BT) la 1-[4-amino-3,5-dibromo-N-[[4-(3,4-dihydro-2(1H)-oxoquinazolin-3-yl)-1-pipéridinyl]carbonyl]-D-phénylalanyl]-4-(4-pipéridinyl)pipéridine,
(BU) la 1-[3,5-dibromo-N-[[4-(1,3-dihydro-4-phényl-2(2H)-oxoimidazol-1-yl)-1-pipéridinyl]carbonyl]-D-tyrosyl]-4-(4-pyridinyl)pipéridine,
(BV) la 1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(1-méthyl-4-pipéridinyl)pipérazine,
(BW) la 1-[N²-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-L-lysyl]-4-(4-pyridinyl)pipérazine,
(BX) la [3,5-dibromo-N-[[4-(1,3-dihydro-4-(3-thiényl)-2(2H)-oxoimidazol-1-yl)-1-pipéridinyl]carbonyl]-D-tyrosyl]-4-(1-pipéridinyl)pipéridine,
(BY) la 1-[4-amino-N-[[4-[4-(3-chlorophényl)-1,3-dihydro-2(2H)-oxoimidazol-1-yl]-1-pipéridinyl]carbonyl]-3,5-dibromo-D-phénylalanyl]-4-(1-méthyl-4-pipéridinyl)pipéridine,
(BZ) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(hexahydro-1H-1-azépinyl)pipéridine,
(CA) la 1-[4-amino-3,5-dibromo-N-[[4-[1,3-dihydro-4-[3-(trifluorométhyl)-phényl]-2(2H)-oxoimidazol-1-yl]-1-pipéridinyl]carbonyl]-D-phénylalanyl]-4-(1-méthyl-4-pipéridinyl)pipérazine,
(CB) la 1-[4-amino-N-[[4-[4-(3-chlorophényl)-1,3-dihydro-2(2H)-oxoimidazol-1-yl]-1-pipéridinyl]carbonyl]-3,5-dibromo-D-phénylalanyl]-4-(hexahydro-1H-1-azépinyl)pipéridine,
(CC) la 1-[4-amino-3,5-dibromo-N-[[4-(1,3-dihydro-4-phényl-2(2H)-oxoimidazol-1-yl)-1-pipéridinyl] carbonyl]-D-phénylalanyl]-4-(4-pyridinyl)pipérazine,
(CD) la 1-[3,5-dibromo-N-[[4-(1,3-dihydro-4-phényl-2(2H)-oxoimidazol-1-yl)-1-pipéridinyl] carbonyl]-D-tyrosyl]-4-(1-méthyl-4-pipéridinyl)pipéridine,
(CE) la 1-[4-amino-3,5-dibromo-N-[[4-[1,3-dihydro-4-phényl-2(2H)-oxoimidazol-1-yl]-1-pipéridinyl]carbonyl]-D-phénylalanyl]-4-[4-(1-oxoéthyl)phényl]pipérazine,
(CF) la 1-[3,5-dibromo-N-[[4-[3,4-dihydro-2(1H)-oxoquinazolin-3-yl]-1-pipéridinyl]carbonyl]-D-tyrosyl]-4-(1-méthyl-4-pipéridinyl)pipérazine,
(CG) la 1-[4-amino-3,5-dibromo-N-[[4-[1,3-dihydro-4-(3-nitrophényl)-2(2H)-oxoimidazol-1-yl]-1-pipéridinyl]carbonyl]-D-phénylalanyl]-4-(1-méthyl-4-pipéridinyl)pipéridine,
(CH) la 1-[4-amino-3,5-dibromo-N-[[4-[3,4-dihydro-2(1H)-oxoquinazolin-3-yl]-1-pipéridinyl]carbonyl]-D-phénylalanyl]-4-(1-pyrrolidinyl)pipéridine,
(CI) la 1-[4-amino-3,5-dibromo-N-[[4-(1,3-dihydro-4-phényl-2(2H)-oxoimidazol-1-yl)-1-pipéridinyl]carbonyl]-D-phénylalanyl]-4-(hexahydro-1H-1-azépinyl)pipéridine et
(CJ) la [4-amino-3,5-dibromo-N-[[4-(1,3-dihydro-4-(3-thiényl)-2(2H)-oxoimidazol-1-yl)-1-pipéridinyl]carbonyl]-D-phénylalanyl]-4-(1-méthyl-4-pipéridinylpipérazine, et
(b) 1,2 à 2 équivalents d'un acide solubilisant physiologiquement acceptable, la substance active étant transformée au moyen de l'acide en le sel correspondant et se trouvant en solution.

2. Composition pharmaceutique selon la revendication 1, comprenant 10 à 20 % en masse d'une substance active du groupe de (A) à (CJ) en solution aqueuse.

3. Composition pharmaceutique selon la revendication 1 ou 2, comprenant 1,6 à 1,9 équivalent d'un acide solubilisant physiologiquement acceptable en solution aqueuse.

4. Composition pharmaceutique selon l'une des revendications 1 à 3, **caractérisée en ce que** l'acide solubilisant physiologiquement acceptable utilisé est choisi dans le groupe constitué par l'acide chlorhydrique, l'acide phosphorique, l'acide méthanesulfonique, l'acide acétique, l'acide formique et l'acide succinique.

5. Composition pharmaceutique selon l'une des revendications 1 à 3, **caractérisée en ce que** l'acide solubilisant physiologiquement acceptable utilisé est l'acide chlorhydrique.

6. Composition pharmaceutique selon l'une des revendications 1 à 5, contenant en outre un ou plusieurs agents facilitant l'absorption à une concentration de 0,1 % à 5 % m/v.

7. Composition pharmaceutique selon l'une des revendications 1 à 5, contenant en outre un agent gélifiant à une concentration de 0,05 % à 1 % m/v pour augmenter la viscosité.

8. Composition pharmaceutique selon l'une des revendications 1 à 5, contenant en outre un phospholipide formateur de liposomes à une concentration de 2 % à 10 % m/v et éventuellement en plus un produit d'hydrolyse d'un phospholipide formateur de liposomes à une concentration de 0,5 % à 1 % m/v.

9. Composition pharmaceutique selon l'une des revendications précédentes, conditionnée sous un gaz protecteur sous forme de doses individuelles dans des unités d'emballage primaire, les unités d'emballage primaire étant contenues dans un emballage secondaire, en forme de sachets ou de sachets tubulaires d'aluminium, de feuille métallisée ou de feuille transparente, sous une atmosphère de gaz protecteur, éventuellement avec un agent fixateur d'oxygène.

10. Composition pharmaceutique selon l'une des revendications précédentes, **caractérisée en ce que** la substance active est
la 1-[N²-[3,5-dibromo-N-[[4-(3,4-dihydro-2(1H)-oxoquinazolin-3-yl)-1-pipéridinyl]-carbonyl]-D-tyrosyl]-L-lysyl]-4-(4-pyridinyl)pipérazine ou
la 1-[4-amino-3,5-dibromo-N-[[4-(2,3,4,5-tétrahydro-2(1H)-oxo-1,3-benzodiazépin-3-yl)-1-pipéridinyl]carbonyl]-D-phénylalanyl]-4-(1-pipéridinyl)pipéridine.

11. Procédé de préparation d'une composition pharmaceutique selon l'une des revendications précédentes, comprenant les étapes suivantes :
(a) dissolution de 2 à 25 % m/v, rapporté à la composition pharmaceutique, d'une substance active choisie dans le groupe de (A) à (CJ) selon la revendication 1 dans une solution aqueuse comprenant 1,2 à 2 équivalents d'un acide solubilisant physiologiquement acceptable, ou
(b) dissolution d'un sel d'une substance active choisie dans le groupe de (A) à (CJ) selon la revendication 1, formé avec un acide solubilisant physiologiquement acceptable, dans de l'eau en une quantité telle que la teneur en substance active de la composition, rapportée à la substance active, soit de 2 % à 25 % m/v, et
(c) addition de 1,2 à 2 équivalents d'un acide solubilisant physiologiquement acceptable et
(d) addition éventuelle d'une ou plusieurs substances auxiliaires choisies parmi des agents facilitant l'absorption, des agents gélifiants et des phospholipides formateurs de liposomes, ainsi que
(e) éventuellement conditionnement de la solution ainsi obtenue sous gaz protecteur sous forme de doses individuelles dans des unités d'emballage primaire, les unités d'emballage primaire étant contenues dans un emballage secondaire, en forme de sachets ou de sachets tubulaires d'aluminium, de feuille métallisée ou de feuille transparente, sous une atmosphère de gaz protecteur, éventuellement avec un agent fixateur d'oxygène.
